(19) Europäisches Patentamt
European Patent Office
Office européen des brevets

(11) EP 3 763 225 A1

(12) EUROPEAN PATENT APPLICATION

(43) Date of publication:
**13.01.2021 Bulletin 2021/02**

(21) Application number: **20182011.5**

(22) Date of filing: **24.06.2020**

(51) Int Cl.:
***A23L 33/10** (2016.01)* ***A23L 33/105** (2016.01)*
***A61P 17/00** (2006.01)* ***A61P 17/18** (2006.01)*
***A23L 7/104** (2016.01)* ***A61K 8/368** (2006.01)*
***A61Q 19/08** (2006.01)* ***A61K 31/05** (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.07.2019 US 201962871336 P**
**15.06.2020 EP 20180003**

(71) Applicant: **TCI Co., Ltd.**
**Taipei 11494 (TW)**

(72) Inventors:
- **HUANG, CHU-HAN**
**11494 Taipei (TW)**
- **CHUNG, YU-MING**
**11494 Taipei (TW)**
- **LIN, YUNG-HSING**
**11494 Taipei (TW)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **BLACK RICE EXTRACT FERMENTS AND THE PREPARATION METHODS AND APPLICATIONS THEREOF**

(57) A composition comprising a fermentation product of black rice extract, uses of the fermentation product and its active ingredients, and a method for manufacturing the fermentation product. The fermentation product of black rice extract comprises at least one of the following compounds of formula (I) to formula (V):

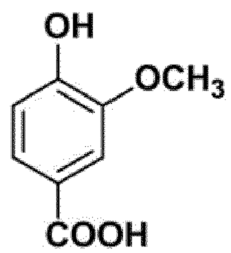 (I), 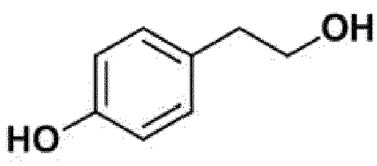 (II), 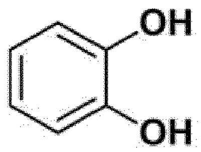 (III),

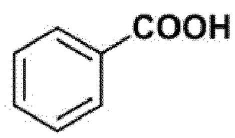 (IV) and 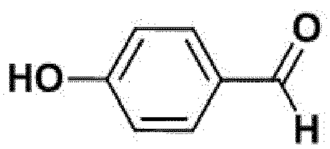 (V).

EP 3 763 225 A1

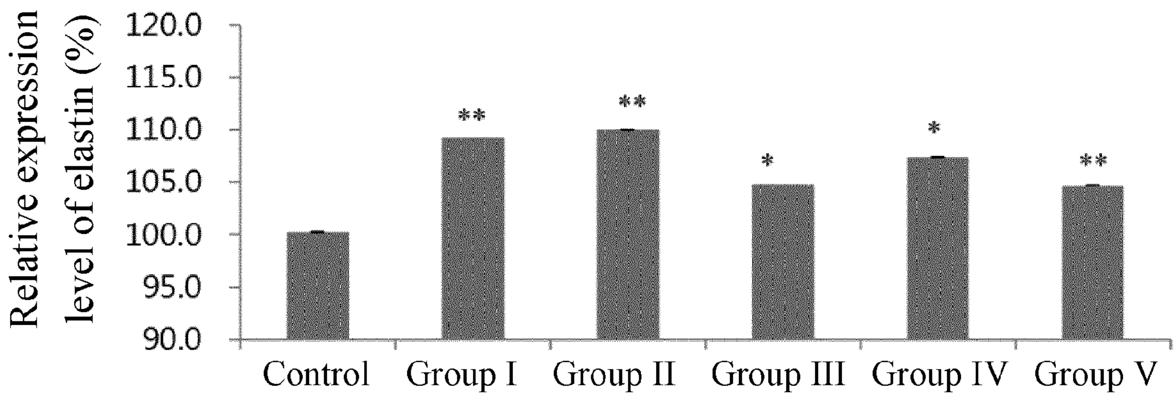
Relative expression level of elastin (%)
120.0
115.0
110.0
105.0
100.0
95.0
90.0
**
**
*
*
**
Control
Group I
Group II
Group III
Group IV
Group V

FIG. 20

## Description

## FIELD OF THE INVENTION

The present invention relates to a composition comprising a fermentation product of black rice extract containing at least one of the following compounds of formula (I) to formula (V) and a method for manufacturing the fermentation product of black rice extract:

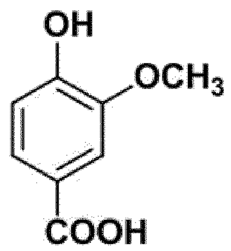
(I),
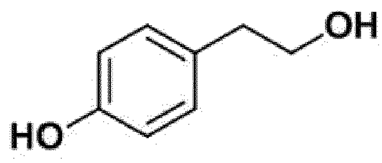
(II),
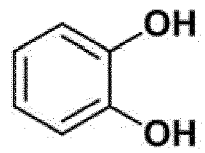
(III),
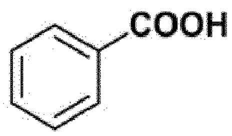
(IV) and
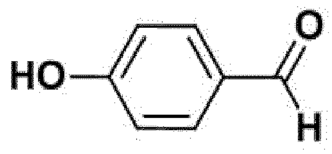
(V).

The present invention also relates to the uses of at least one of the fermentation product of black rice extract and the compounds of formula (I) to formula (V), especially relates to the uses in inhibiting skin aging, moisturizing skin, reducing wrinkles, enhancing skin elasticity, tightening skin, inhibiting the production of advanced glycation end product (AGEs), inhibiting oxidation, enhancing expression of aquaporin, enhancing the secretion of collagen and enhancing expression of elastin.

## BACKGROUND OF THE INVENTION

Skin, which is the biggest organ in the human body as well as the first line of defense against foreign invasion and external environmental stimuli, has functions in water retention, warm retention, sensation, etc. When skin is affected by ultraviolet (UV) rays, radiation, microorganisms and particulate matter in the environment, the water content of skin may decrease, and the aging of skin cells may accelerate, thereby causing the occurrence of phenomena such as thickening of the horny layer (stratum corneum), desiccation and desquamation of skin, generation of fine lines and dark spots, skin sagging, and degeneration and loss of skin proteins, and may even damage the DNA of skin cells that may induce cytotoxicity or lead to skin cell variation, and thus, result in a skin lesion.

It is known that increasing the expression of aquaporin helps enhance the moisturizing ability of the skin. Therefore, if the expression of aquaporin can be enhanced, the effect of moisturizing can be achieved. Also, collagen, which is also an important substrate for increasing the water content of the skin, not only serves the function of retaining water but also imparts resilience and elasticity of the skin. Elastin in the dermis is capable of supporting the structure of skin cells. Therefore, if the contents of collagen and elastin in the skin can be increased, the effects of enhancing skin elasticity, tightening skin and reducing wrinkles can be achieved.

Glycosylation is a chemical reaction in which glucose is attached to a protein and advanced glycation end product (AGEs) are thus generated. The AGEs accumulated in the skin cells may not only lead to protein denaturation and loss of protein elasticity, thereby resulting in skin wrinkle generation and skin aging, but also lead to DNA damage of skin cells, affect DNA function of skin cells and even cause skin lesions. Therefore, if AGEs can be inhibited, the effects of tightening skin, enhancing skin elasticity, reducing wrinkles, and inhibiting skin aging can be achieved.

In recent years, people pay much more attention to skincare and prefer to use natural and safe materials in their skincare products. Therefore, the industry keeps developing effective approaches for skincare that use natural and safe materials. Inventors of the present invention found that the fermentation product of black rice extract, which is provided by extracting black rice and further fermenting the obtained extract, can inhibit the production of AGEs, inhibit oxidation, enhance expression of aquaporin, enhance secretion of collagen and enhance expression of elastin, and thus can be used in skincare to inhibit skin aging, moisturize skin, reduce wrinkles, enhance skin elasticity and/or tighten skin.

## SUMMARY OF THE INVENTION

Therefore, an objective of the present invention is to provide a method for manufacturing a fermentation product of black rice extract, comprising the following steps: (a) extracting black rice by using a polar solvent to provide a black rice extract; (b) fermenting the extract by using *Saccharomyces cerevisiae* to provide an intermediate fermentation product; and (c) fermenting the intermediate fermentation product by using *Lactobacillus plantarum* to provide a fermentation product of black rice extract. Preferably, the polar solvent is water, alcohol, or a combination thereof, an amylase is used in step (a), the *Saccharomyces cerevisiae* is *Saccharomyces cerevisiae* BCRC 20271, and the *Lactobacillus plantarum* is *Lactobacillus plantarum* BCRC 910805.

Still another objective of the present invention is to provide a composition, comprising a fermentation product of black rice extract provided by the above method. Preferably, the fermentation product of black rice extract comprises at least one of the following compounds of formula (I) to formula (V):

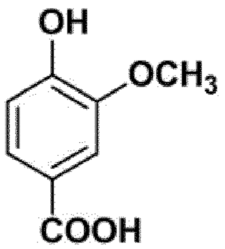 (I), 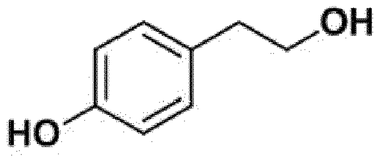 (II), 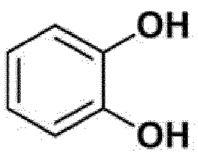 (III),

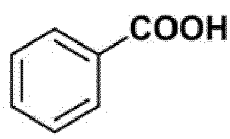 (IV) and 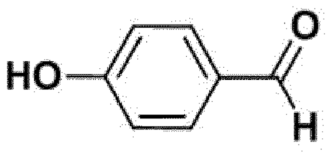 (V).

Preferably, the composition is a pharmaceutical composition, a food composition or a care product composition. More preferably, the food composition is a health food, a nutritional supplement or a special nutritional food, and the pharmaceutical composition is formulated for oral administration, injection or transdermal administration.

Yet another objective of the present invention is to provide a use of an active ingredient in inhibiting skin aging, moisturizing skin, reducing wrinkles, enhancing skin elasticity and/or tightening skin, wherein the active ingredient is at least one of the fermentation product of black rice extract provided by the above method and the above compounds of formula (I) to formula (V). Preferably, the active ingredient is used in a form of a food composition or a care product composition.

Yet another objective of the present invention is to provide a use of an active ingredient in the manufacture of a pharmaceutical composition, wherein the pharmaceutical composition is for use in at least one of inhibiting production of AGEs, inhibiting oxidation, enhancing expression of aquaporin, enhancing secretion of collagen and enhancing expression of elastin, and the active ingredient is at least one of the fermentation product of black rice extract provided by the above method and the above compounds of formula (I) to formula (V).

Yet another objective of the present invention is to provide a composition for use in inhibiting skin aging, moisturizing skin, reducing wrinkles, enhancing skin elasticity and/or tightening skin, characterized in that the composition comprises an effective amount of an active ingredient, and the active ingredient is at least one of the fermentation product of black rice extract provided by the above method and the above compounds of formula (I) to formula (V). Preferably, the composition is a food composition or a care product composition. More preferably, the food composition is a health food, a nutritional supplement or a special nutritional food.

Yet another objective of the present invention is to provide a pharmaceutical composition for use in at least one of inhibiting production of AGEs, inhibiting oxidation, enhancing expression of aquaporin, enhancing secretion of collagen and enhancing expression of elastin, characterized in that the composition comprises an effective amount of an active ingredient, and the active ingredient is at least one of the fermentation product of black rice extract provided by the above method and the above compounds of formula (I) to formula (V). Preferably, the pharmaceutical composition is formulated for oral administration, injection or transdermal administration.

## BRIEF DESCRIPTION OF THE DRAWINGS

**FIGs. 1A** to **1E** respectively shows the NMR spectrums of compounds of formula (I) to formula (V).

**FIG. 2** shows the SOD activity each of the "black rice extract group" and "black rice fermentation product group," wherein the reaction solution of the "black rice extract group" contained a black rice extract, and the reaction solution of the "black rice fermentation product group" contained a fermentation product of black rice extract.

**FIG. 3** shows the SOD activity each of the "white rice fermentation product group" and "black rice fermentation product group," wherein the reaction solution of the "white rice fermentation product group" contained a fermentation product of white rice extract, and the reaction solution of the "black rice fermentation product group" contained a fermentation product of black rice extract.

**FIG. 4** shows the glycosylation-inhibitory activity each of the "black rice extract group" and "black rice fermentation product group," wherein the reaction solution of the "black rice extract group" contained a black rice extract, and the reaction solution of the "black rice fermentation product group" contained a fermentation product of black rice extract.

**FIG. 5** shows the glycosylation-inhibitory activity each of the "white rice fermentation product group" and "black rice fermentation product group," wherein the reaction solution of the "white rice fermentation product group" contained a fermentation product of white rice extract, and the reaction solution of the "black rice fermentation product group" contained a fermentation product of black rice extract.

**FIG. 6** shows the content of moisturizing polysaccharide each of the "black rice extract group" and "black rice fermentation product group," wherein the reaction solution of the "black rice extract group" contained a black rice extract, and the reaction solution of the "black rice fermentation product group" contained a fermentation product of black rice extract.

**FIG. 7** shows the content of moisturizing polysaccharide each of the "white rice fermentation product group" and "black rice fermentation product group," wherein the reaction solution of the "white rice fermentation product group" contained a fermentation product of white rice extract, and the reaction solution of the "black rice fermentation product group" contained a fermentation product of black rice extract.

**FIG. 8** shows the photos of stained human primary epidermal keratinocytes (the portion with blue fluorescence was nucleus; the portion with green fluorescence was aquaporin; scale bar represents 100$\mu$m) that were taken under microscope, wherein the photos include the results of the "control group," "black rice extract group" and "black rice fermentation product group," and wherein, the human primary epidermal keratinocytes of the "control group" were cultured in a medium free of the black rice extract and fermentation product of black rice extract, the human primary epidermal keratinocytes of the "black rice extract group" were cultured in a medium containing a black rice extract, and the human primary epidermal keratinocytes of the "black rice fermentation product group" were cultured in a medium containing a fermentation product of black rice extract.

**FIG. 9** are photos taken by camera, showing the collagen gels of "control group," "black rice extract group" and "black rice fermentation product group" (the yellow line was the outline of the collagen gel of "control group," the blue line was the outline of the collagen gel of "black rice extract group" and the red line was the outline of the collagen gel of "black rice fermentation product group"), wherein the collagen gel of "control group" was treated with a medium free of the black rice extract and fermentation product of black rice extract, the collagen gel of the "black rice extract group" was treated with a medium containing a black rice extract, and the collagen gel of the "black rice fermentation product group" was treated with a medium containing a fermentation product of black rice extract.

**FIG. 10** shows that in comparison with the control group, the relative collagen densities of the collagen gels of "black rice extract group" and "black rice fermentation product group" (** indicates that the result has a significant difference to that of the control group with a p value<0.01).

**FIGs. 11** to **13** respectively shows the effects of the fermentation product of black rice extract of the present invention, which was applied on the skin, enhancing the moisture of the skin, reducing skin sagging and enhancing skin elasticity, including the results of "placebo (a mask free of a fermentation product of black rice extract)" and "black rice fermentation product (a mask containing a fermentation product of black rice extract)."

**FIGs. 14** to **17** respectively shows the effects of the fermentation product of black rice extract of the present invention, which was administered orally, enhancing the moisture of the skin, enhancing skin elasticity, reducing skin sagging, and reducing skin wrinkles, including the result of "week 0 (prior to drinking the beverage containing a fermentation product of black rice extract of the present invention)" and "week 4 (after drinking the beverage containing a fermentation product of black rice extract of the present invention)" (* indicates that the result has a significant difference to that of week 0 with a p value<0.05).

**FIG. 18** are photos taken by camera, showing skin wrinkles of two subjects, including the results of "week 0 (prior to drinking the beverage containing a fermentation product of black rice extract of the present invention)" and "week

4 (after drinking the beverage containing a fermentation product of black rice extract of the present invention)."

**FIG. 19** shows the relative secretion level of collagen in human skin fibroblast each of the "control group," "group I," "group II," group III," "group IV" and "group V," wherein the cells of "control group" were cultured in a medium free of the compounds of the present invention, and the cells of "group I" to "group V" were respectively cultured in medium that contains compound of formula (I), compound of formula (II), compound of formula (III), compound of formula (IV) or compound of formula (V) (* indicates that the result has a significant difference to that of the control group with a p value<0.05; ** indicates that the result has a significant difference to that of the control group with a p value<0.01).

**FIG. 20** shows the relative expression level of elastin in human skin fibroblast each of the "control group," "group I," "group II," group III," "group IV" and "group V," wherein the cells of "control group" were cultured in a medium free of the compounds of the present invention, and the cells of "group I" to "group V" were respectively cultured in medium that contains compound of formula (I), compound of formula (II), compound of formula (III), compound of formula (IV) or compound of formula (V) (* indicates that the result has a significant difference to that of the control group with a p value<0.05; ** indicates that the result has a significant difference to that of the control group with a p value<0.01).

**FIG. 21** shows the HPLC fingerprint spectrum each of the "fermentation product of black rice extract" and "black rice extract," wherein the top figure shows the result of the fermentation product of black rice extract, and the bottom figure shows the result of the black rice extract.

## DESCRIPTION OF THE PREFERRED EMBODIMENTS

The following paragraphs will describe some of the embodiments of the present invention in detail. However, without departing from the spirit of the present invention, the present invention may be embodied in various embodiments and should not be limited to the embodiments described in the specification or defined in the appended claims.

Unless otherwise indicated herein, the expression "a," "an," "the," or the like recited in the specification of the present invention (especially in the claims) are intended to include both the singular and plural forms; the term "subject" recited herein refers to human or non-human mammalian (e.g., dog, cat).

Black rice, also called black kernelled rice, is a kind of indica rice with a black and purple appearance. It is rich in nutrients such as anthocyanin, calcium, phosphorus, iron and vitamin B1. Black rice belongs to a form of low GI (Glycemic index) food, and thus, is suitable for a diabetic patient.

The term "amylase" recited herein refers to any amylase that is suitable for the present invention, including amylase that can be purchased by the general public. Besides, the *"Saccharomyces cerevisiae"* and *"Lactobacillus plantarum"* recited herein can also be any one that is suitable for the present invention, including *Saccharomyces cerevisiae* and *Lactobacillus plantarum* that can be purchased by the general public (e.g., domestic or international deposit institution) and *Saccharomyces cerevisiae* and *Lactobacillus plantarum* being isolated from natural sources by using the conventional microorganism isolating methods in the art.

Inventors of the present invention found that as compared to an unfermented black rice extract, the fermentation product provided by fermenting a black rice extract in the presence of *Saccharomyces cerevisiae* and *Lactobacillus plantarum* has better effects on inhibiting production of AGEs, inhibiting oxidation, enhancing expression of aquaporin, and tightening the skin. Therefore, the fermentation product provided by black rice extract provided according to the present invention (i.e., fermentation product of black rice extract) can be used in inhibiting skin aging, moisturizing the skin, reducing wrinkles, enhancing elasticity, tightening skin.

Inventors of the present invention further obtained the following five active ingredients (i.e., compounds of formula (I) to formula (V)) from the above fermentation product of black rice extract. Those active ingredients all can enhance the secretion of collagen and enhance the expression of elastin:

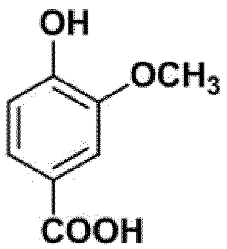 (I), 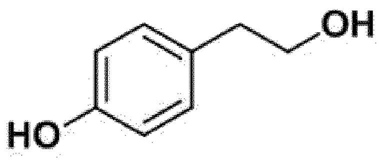 (II), 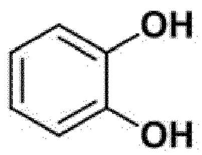 (III),

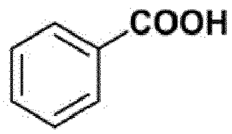 (IV) and 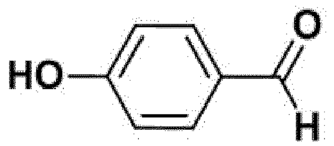 (V).

Therefore, the present invention relates to a method for manufacturing a fermentation product of black rice extract; a composition comprising at least one of the fermentation product of black rice extract provided by the above method and compounds of formula (I) to formula (V); a use of at least one of the fermentation product of black rice extract provided by the above method and compounds of formula (I) to formula (V) in inhibiting skin aging, moisturizing the skin, reducing wrinkles, enhancing skin elasticity and/or tightening skin; and a use of at least one of the fermentation product of black rice extract provided by the above method and compounds of formula (I) to formula (V) in the manufacture of a pharmaceutical composition for at least one of inhabiting production of AGEs, inhibiting oxidation, enhancing expression of aquaporin, enhancing secretion of collagen and enhancing expression of elastin.

The method of manufacturing a fermentation product of black rice extract of the present invention comprises the following steps: (a) extracting black rice by using a polar solvent to provide a black rice extract; (b) fermenting the extract by using *Saccharomyces cerevisiae* to provide an intermediate fermentation product; and (c) fermenting the intermediate fermentation product by using *Lactobacillus plantarum* to provide a fermentation product of black rice extract.

In step (a), the polar solvent can be used for conducting the extraction. Preferably, the polar solvent is water, alcohol (e.g., C1-C4 alcohol), or a combination thereof. In some embodiments of the present invention, water was adopted as the extracting solvent. Generally, the amount of extracting solvent can be optionally adjusted, and there is no particular limitation to the amount of extracting solvent as long as the material can be evenly dispersed in the extracting solvent. For example, in step (a), the black rice and extracting solvent can be used at a weight ratio ranging from 1: 3 to 1: 9 (black rice: extracting solvent). Furthermore, in step (a), the extraction can be conducted at a suitable temperature for a suitable period of time depending on the extracting solvent that is adopted. For example, when the extracting solvent is water and the weight ratio of black rice and extracting solvent is ranging from 1: 3 to 1: 9 (black rice: extracting solvent), the extraction is usually conducted for 1.5 hours at 95°C.

In step (a), amylase(s) is optionally added into the mixture of the extracting solvent and black rice before conducting the extraction. In general, there is no particular limitation to the amount of amylase as long as the starch in the black rice can be degenerated into saccharides. For example, in step (a), the "amylase" and the "mixture of the extracting solvent and black rice" can be used at a weight ratio ranging from 1: 100 to 1: 200 (amylase: mixture).

In step (b), *Saccharomyces cerevisiae* is added into the extract provided by step (a) to conduct the first fermentation. In some embodiments of the present invention, *Saccharomyces cerevisiae* BCRC 20271 was adopted to ferment the extract provided by step (a). Furthermore, in step (b), the fermentation can be conducted at a suitable temperature for a suitable period of time depending on the adopted *Saccharomyces cerevisiae*. For example, when *Saccharomyces cerevisiae* BCRC 20271 is used, the fermentation is usually conducted at room temperature for 20 to 24 hours. In general, there is no particular limitation to the amount of *Saccharomyces cerevisiae.* For example, in step (b), the amount of *Saccharomyces cerevisiae* can be $8 \times 10^5$ to $1.2 \times 10^6$ cfu per gram (g) of the extract.

In step (c), *Lactobacillus plantarum* is added into the intermediate fermentation product provided by step (b) to conduct the second fermentation. In some embodiments of the present invention, *Lactobacillus plantarum* BCRC 910805 was adopted to ferment the intermediate fermentation product provided by step (b). Furthermore, in step (c), the fermentation can be conducted at a suitable temperature for a suitable period of time depending on the adopted *Lactobacillus plantarum.* For example, when *Lactobacillus plantarum* BCRC 910805 is used, the fermentation is usually conducted at room temperature for 1 to 3 days. In general, there is no particular limitation to the amount of *Lactobacillus plantarum.* For example, in step (c), the amount of *Lactobacillus plantarum* can be $8 \times 10^6$ to $5.0 \times 10^7$ cfu per gram (g) of the intermediate fermentation product.

Without removing the aforementioned *Saccharomyces cerevisiae* and *Lactobacillus plantarum* contained in the fermentation product, the fermentation product of black rice extract provided by step (c) usually complies the standards of sugar degree < 4°, pH value = 2-4, and alcohol > 5%.

After accomplishing the above steps, the fermentation product of black rice extract may be optionally vacuum concentrated, filtrated and sterilized, to further enhance the convenience of using the fermentation product of black rice extract. For example, the fermentation product of black rice extract can be vacuum concentrated at 45°C to 70°C, to provide a concentrated fermentation product. Alternatively, the fermentation product of black rice extract or concentrated fermentation product can be filtrated by using a sieve with 200 to 400 meshes, to remove the solid residues. On the other hand, the fermentation product of black rice extract can be added with 40% to 70% (wt./wt.) isomalto-oligosaccharides, and sterilized at 90°C to 120°C for 70 to 90 minutes to provide a beverage.

The pharmaceutical composition provided in accordance with the present invention can be used for systemic

or topical administration and can be delivered by various drug delivery systems (DDSs), such as an oral drug delivery system, transdermal drug delivery system, and injectable drug delivery system. For example, to enhance bioavailability, control drug release speed, target the lesion precisely and reduce side effects, the pharmaceutical composition can be delivered by a liposome, a microcapsule, nanoparticles, or microneedles, but is not limited thereby.

Depending on the desired purpose(s), the pharmaceutical composition of the present invention can be provided in any suitable form without particular limitations. For example, the pharmaceutical composition can be provided in a form for oral administration, intravenous injection (including drip infusion and bolus injection), intramuscular injection, subcutaneous injection, intraarterial injection, intraperitoneal injection or transdermal administration (such as patch and ointment), but is not limited thereby. Depending on the form and purpose(s), a suitable carrier can be chosen and used to provide the pharmaceutical composition, wherein the carriers are well known by a person skilled in the art of pharmaceutical engineering. Examples of the carrier include excipients, diluents, auxiliaries, stabilizers, absorption enhancers, disintegrating agent, hydrotropic agents, emulsifiers, antioxidants, adhesives, binders, tackifiers, dispersants, suspending agents, lubricants, and hygroscopic agents.

As a form for oral administration, the pharmaceutical composition can be provided by any suitable methods in any suitable form for oral administration, wherein the suitable liquid form for oral administration includes syrups, an oral solution, a suspension and an elixir, and the suitable solid form for oral administration includes a powder, a granule, a troche, a dragee, an enteric-coated tablet, a chewable tablet, an effervescent tablet, a film-coated tablet, a capsule and a long-acting slow-release tablet. The pharmaceutical composition provided in accordance with the present invention can comprise any pharmaceutically acceptable carrier that will not adversely affect the desired effects of the active ingredient (i.e., at least one of the fermentation product of black rice extract and compounds of formula (I) to formula (V)). For example, the pharmaceutically acceptable carriers for the aforesaid liquid form include, but are not limited to, water, saline, dextrose, glycerol, ethanol or its analogs, oil (e.g., olive oil, castor oil, cottonseed oil, peanut oil, corn oil, and germ oil), glycerol, polyethylene glycol, and combinations thereof; and the pharmaceutically acceptable carriers for the aforesaid solid form include, but are not limited to, cellulose, starch, kaolinite, bentonite, sodium citrate, gelatin, agar, carboxymethyl cellulose, gum arabic, seaweed gel, glyceryl monostearate, calcium stearate, and combinations thereof.

As a form for transdermal administration, the pharmaceutical composition of the present invention can also comprise any pharmaceutically acceptable carrier that will not adversely affect the desired effects of the active ingredient (i.e., at least one of the fermentation product of black rice extract and compounds of formula (I) to formula (V)), such as water, mineral oil, propylene glycol, polyethylene oxide, liquid petrolatum, sorbitan monostearate, and polysorbate 60. The pharmaceutical composition can be provided by any suitable methods in any suitable form for transdermal administration, such as in the form of an emulsion, a cream, an oil, a gel (such as a hydrogel), a paste (such as a dispersing paste and an ointment), a lotion, a spray and a patch (such as a microneedle patch), but is not limited thereby.

As for the form of injections or drips, the pharmaceutical composition can comprise one or more ingredient(s), such as an isotonic solution, a salt-buffered saline (e.g., phosphate-buffered saline or citrate-buffered saline), a hydrotropic agent, an emulsifier, a 5% sugar solution, and other carriers to provide the pharmaceutical composition as an intravenous infusion, an emulsified intravenous infusion, a powder for injection, a suspension for injection or a powder suspension for injection. Alternatively, the pharmaceutical composition can be prepared as a pre-injection solid. The desired injection is provided by dissolving the pre-injection solid in other solutions or suspensions or emulsifying it before being administered to a subject in need.

The food composition provided in accordance with the present invention can be a beverage, a portion of solid food or semi-solid food, and can be provided in the form of a daily supplement, a health food, a functional food, a nutritional supplement or special nutritional food. For example, the food composition can be manufactured as dairy products, meat products, bread, pasta, cookies, frozen desserts, troche, capsule, fruit juices, teas, sparkling water, alcoholic drinks, sports drinks, nutritional drinks, but is not limited thereby. Preferably, the food composition is provided in the form of a daily supplement or health food.

In addition, depending on the form and needs, the food composition in accordance with the present invention can comprise any suitable food additives. For example, the food additives include, but are not limited to, a preservative, a bactericide, an antioxidant, a bleaching agent, a color fasting agent, a swelling agent, a nutrition additive, a colorant, a flavoring agent (e.g., sweetener), a pasting agent, a binding agent, chemicals for food industry, an emulsifier, and agents for improving quality, brewing and food manufacturing.

The recommended daily dosage, use standards and use conditions for a specific population (e.g., pregnant woman and children), or the recommendations for a use in combination with another food product or medicament can be indicated on the exterior package of the daily supplement, health food, functional food, nutritional supplement or special nutritional food provided in accordance with the present invention. Thus, it is suitable for the user to take the daily supplement, health food, functional food, nutritional supplement or special nutritional food by an individual safely and securely without the instructions of a doctor, pharmacist, or related executive.

The care product composition provided in accordance with the present invention can be produced in any suitable form For example, the care product composition can be a skin care product such as softening toner, convergent toner,

nutrition toner, nutritional cream, massage cream, essence, eye cream, eye essence, facial mask, patch, spray, body lotion, body cream, body oil and body essence; a makeup product such as makeup primer, foundation, face powder, blush, eyeshadow, brow powder, mascara and lipstick; or a cleaning product such as cleaning oil, cleaning cream, cleaning lotion, eye and lip makeup remover, soap and body wash, but is not limited thereby. In addition, the care product composition can also be used in a foam form or an aerosol form that further contains a compressed propellant.

Depending on the form and needs of the product, the care product composition provided in accordance with the present invention can comprise any cosmetically or dermatologically acceptable vehicles that will not adversely affect the desired effects of the active ingredient (i.e., at least one of the fermentation product of black rice extract and compounds of formula (I) to formula (V)). For example, when the care product composition is an emulsion, cream or gel product, ingredients such as wax, paraffin, starch, tragacanth gum, cellulose derivatives, animal oil, plant oil, mineral oil, polyethylene glycol, bentonite, silicon oxide, talc and zinc oxide can be used as the vehicle; when the care product composition is a powder or aerosol product, ingredients such as lactose, talc, silicon oxide, aluminum hydroxide, calcium silicate and polyamide can be used as the vehicle; when the care product composition is a solution or emulsion product, ingredients such as water, ethanol, isopropanol, ethyl carbonate, ethyl acetate, benzyl alcohol, benzyl benzoate, propylene glycol and polyethylene glycol can be used as the vehicle, but is not limited thereby.

When the care product composition is provided in the form of a cleaning product, it can further comprise any surfactants that will not adversely affect the desired effects of the active ingredients (i.e., at least one of the fermentation product of black rice extract and compounds of formula (I) to formula (V)) to be the vehicle, wherein examples of the surfactants include, but are not limited to, fatty acid salts, alkylbenzene sulfonate (ABS), fatty alcohol sulfate (FAS), alcohol ether sulfate (AES), fatty alcohol polyoxyethylene ether carboxylate (AEC), fatty acid methyl ester sulfonate (MES), fatty alcohol ethoxylates (AE), alkyl polyglycoside (APG), betaine type surfactant, and combinations thereof.

Optionally, the pharmaceutical composition, food composition or care product composition provided in accordance with the present invention can also comprise a suitable amount of additives, such as a toner or a colorant for enhancing the visual perception of the pharmaceutical composition, food composition or care product composition, and/or a buffer, a conservative, a preservative, an antibacterial agent, or an antifungal agent for improving the stability and storability of the pharmaceutical composition, food composition or care product composition.

The pharmaceutical composition, food composition or care product composition provided in accordance with the present invention can optionally further comprise one or more other active ingredient(s) (such as collagen, hyaluronic acid, amino acid, vitamin A, glycerol and lactic acid) to further enhance the effects of the pharmaceutical composition, food composition or care product composition, or to increase the application flexibility and application adaptability of preparation thus provided, as long as the other active ingredients do not adversely affect the desired effects of the active ingredient of the present invention (i.e., at least one of the fermentation product of black rice extract and compounds of formula (I) to formula (V)).

The pharmaceutical composition, food composition or care product composition provided in accordance with the present invention contains at least about 0.0001, 0.0002, 0.0003, 0.0004, 0.0005, 0.001, 0.0015, 0.002, 0.0025, 0.003, 0.0035, 0.004, 0.0045, 0.005, 0.0055, 0.006, 0.0065, 0.007, 0.0075, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 5, 10, 15, 20, 25, 30, 35, 40, 45, 50, 55, 60, 65, 70, 75, 80, 85, 90, 95 or 100 wt% of the active ingredient (i.e., at least one of the fermentation product of black rice extract and compounds of formula (I) to formula (V)) based on the total weight of the composition, and the amount of the active ingredient in the composition can be selected from any two of the aforesaid values, for example, about 0.0001 wt% to about 90 wt%, about 0.001 wt% to about 25 wt%, about 0.01 wt% to about 10 wt%, about 0.01 wt% to about 5 wt%, about 0.05 wt% to about 1 wt%, and about 0.05 wt% to about 0.5 wt%.

Depending on the need, age, body weight and health conditions of the subject and the purpose of application, the pharmaceutical composition, food composition or care product composition provided in accordance with the present invention can be taken at various frequencies, such as once a day, multiple times a day, or once every few days. The amount of the active ingredient (i.e., at least one of the fermentation product of black rice extract and compounds of formula (I) to formula (V)) in the pharmaceutical composition, food composition or care product composition provided in accordance with the present invention can be adjusted, for example, the amount can be adjusted to that it should be taken or external used daily, depending on the need in the practical application. Generally, when the composition of the present invention is used in a form of a pharmaceutical composition, the recommended daily dosage is 5 g to 10 g (or 5 ml to 10 ml) fermentation product of black rice extract, or 0.001 mg/kg-body weight of the compounds of the present invention (i.e., at least one of compounds of formula (I) to formula (V)). When the composition of the present invention is used in a form of a food composition, the recommended daily dosage is 5 ml fermentation product of black rice extract, or 0.001 mg/kg-body weight of the compounds of the present invention. Furthermore, when the composition of the present invention is used in a form of a care product composition, the recommended amount contained in the care product composition is 1% fermentation product of black rice extract.

The present invention will be further illustrated in detail with specific examples as follows. However, the following examples are provided only for illustrating the present invention and the scope of the present invention is not limited

thereby. The scope of the present invention will be indicated in the appended claims.

**Example**

**[Preparation example]**

Materials and equipment used in the following examples are described as below:

1. Nuclear Magnetic Resonance Spectrometer (NMR): 400MHz Varian 400 FT-NMR was used for 1D and 2D spectrums; δ represents chemical shift, and its unit is ppm.
2. Mass Spectrometer (MS): LTQ-FT mass spectrometer, using Bruker amaZon SL system for detection, and its unit is m/z.
3. Medium pressure liquid chromatography (MPLC): CombiFlash® Rf+, Teledyne ISCO, Lincoln, NE.
4. High Performance Liquid Chromatography (HPLC):

   (1) Pump system: Hitachi L-2310 series pump;
   (2) Detector: Hitachi L-2420 UV-VIS detector, and the detection wavelength is 200 to 380 nm;
   (3) Data management software: D-2000 Elite software;
   (4) Analyzing column: Discovery® HS C18 (SUPELCO, 250 x 4.6 mm, 5μm);
   (5) Analyzing column: Mightysil RP-18 GP 250 (Kanto, 250 x 4.6 mm, 5μm);
   (6) Semi-preparation column: Discovery® HS C18 (SUPELCO, 250 x 10.0 mm, 5μm); and
   (7) Preparative column: Discovery® HS C18 (SUPELCO, 250 x 21.0 mm, 5μm).

5. Packing material for column chromatography:

   (1) Sephadex LH-20 (Amersham Biosciences);
   (2) Diaion HP-20 (Mitsubishi Chemical);
   (3) Merck Kieselgel 60 (40-63 μm, Art. 9385); and
   (4) Merck LiChroprep® RP-18 (40-63μm, Art. 0250).

6. Thin-Layer Chromatography (TLC): TLC aluminum sheets (silica gel 60 F254, 0.25 mm, purchased from Merck, Germany).
7. UV Lamp: UVP UVGL-25, and the wavelengths are 254 nm and 365 nm.
8. Solvent (purchased from Merck Taiwan): (n-hexane), ethyl acetate, acetone, methanol, ethanol, n-butanol, acetonitrile, chloroform-dl (deuteration degree 99.5%), methanol-d6 (deuteration degree 99.5%), deuterium oxide (deuteration degree > 99.8%), and Dimethyl sulfoxide-d6 (deuteration degree > 99.9%).

**A. Preparation of fermentation product of black rice extract**

**A-1.** Black rice (purchased from Chaio lien foods, Taiwan) was mixed with water at a weight ratio of 1: 6 (black rice: water) to provide a mixture. Thereafter, the mixture was added with 1% (wt./wt.) of amylase and extracted at 95°C for 1.5 hours to provide a black rice extract. The black rice extract was then cooled down to room temperature for use in the following fermentation steps.
**A-2.** The black rice extract provided by A-1 was added with *Saccharomyces cerevisiae* BCRC 20271 (adding amount: 1 x $10^6$ cfu *Saccharomyces cerevisiae* BCRC 20271 per gram of the extract), and was then fermented at room temperature for 24 hours to provide an intermediate fermentation product.
**A-3.** The intermediate fermentation product provided by A-2 was added with *Lactobacillus plantarum* BCRC 910805 (adding amount: 5 x $10^7$ cfu *Lactobacillus plantarum* BCRC 910805 per gram of the intermediate fermentation product), and was then fermented for at room temperature 1.5 days to provide a fermentation product of black rice extract.
**A-4.** The fermentation product of black rice extract provided by A-3, from which the *Saccharomyces cerevisiae* and *Lactobacillus plantarum* were not removed, was subject to an inspection to see whether or not the fermentation product complied with the standards of sugar degree < 4°, pH value = 2-4, and alcohol > 5%. If the previously mentioned standard was satisfied, the fermentation had completed. After completing the aforementioned inspection, the fermentation product was vacuum concentrated at 60°C to provide a concentrated fermentation product. Thereafter, the concentrated fermentation product was filtrated by using a sieve with 200 meshes to remove the solid

residues, and then, was sterilized at 95°C for 90 minutes.

**B. Preparation of fermentation product of white rice extract**

This preparation example was conducted by referring to the method and steps described in "Preparation example A," wherein only the black rice used in the "Preparation example A" was replaced with white rice (purchased from Chaio lien foods, Taiwan) to provide a fermentation product of white rice extract.

**C. Preparation of compounds of formula (I) to formula (V)**

**C-1.** 10 L of the fermentation product of black rice extract provided by A-4 was extracted by liquid partition (using 1: 1 of n-BuOH and water) for three times. The liquid of n-BuOH layer and the liquid of water layer obtained from the three extractions were respectively combined and dried with vacuum concentration. An extract of n-BuOH layer (10.4 g in total) and an extract of water layer (1053.5 g in total) were thus obtained.

**C-2.** The extract of n-BuOH layer (10.4 g in total) provided by C-1 was chromatographed by using Diaion HP-20 as the chromatography material (water was used as the starting eluent, and then the ratio of methanol contained therein was gradually increased to decrease the polar of the eluent). A first fraction (F1), second fraction (F2) and third fraction (F3) were thus obtained.

**C-3.** The second fraction (F2) provided by C-2 was chromatographed via Medium pressure liquid chromatography (MPLC) (a mixture of water and methanol was used as the eluent, wherein water: methanol = 9: 1). Thereafter, a TLC aluminum sheet (silica gel 60 F254, 0.25 mm) was used to combine the obtained fractions, and thus, five sub-fractions (F2-1 to F2-5) were obtained. Sub-fraction F2-3 was chromatographed via silica gel column chromatography (a mixture of dichloromethane and methanol was used as the eluent, wherein dichloromethane: methanol = 9: 1). Thereafter, a TLC aluminum sheet (silica gel 60 F254, 0.25 mm) was used to combine the obtained fractions, and thus, five sub-fractions (F2-3-1 to F2-3-5) were obtained, wherein F2-3-2 was the compound of formula (II) (15.2 mg). On the other hand, sub-fraction F2-2 was chromatographed via silica gel column chromatography (a mixture of dichloromethane and methanol was used as the eluent, wherein dichloromethane: methanol = 12: 1), and thus, compound of formula (III) was obtained (10.3 mg).

**C-4.** The third fraction (F3) provided by C-2 was chromatographed via silica gel column chromatography (a mixture of dichloromethane and methanol was used as the eluent, wherein dichloromethane: methanol = 12: 1), and thus, six sub-fractions (F3-1 to F3-6) were obtained. Sub-fraction F3-1 was chromatographed via silica gel column chromatography (a mixture of n-hexane and ethyl acetate was used as the eluent, wherein n-hexane: ethyl acetate = 2: 1). Thereafter, a TLC aluminum sheet (silica gel 60 F254, 0.25 mm) was used to combine the obtained fractions, and thus, compound of formula (V) (5.2 mg) was obtained. On the other hand, sub-fraction F3-2 was chromatographed via silica gel column chromatography (a mixture of n-hexane and ethyl acetate was used as the eluent, wherein n-hexane: ethyl acetate = 2: 1), and thus, compound of formula (I) (3.5 mg) and compound of formula (IV) (6.8 mg) were obtained.

**C-5.** The compounds of formula (I) to formula (V) were analyzed via Nuclear Magnetic Resonance Spectrometer and Mass Spectrometer. After being compared with the references, the chemical structures of compounds of formula (I) to formula (V) were confirmed and listed in the following Table 1, and the NMR spectrums thereof are shown in Figures 1A to IE. The chemical names of compounds of formula (I) to formula (V) are vanillic acid, tyrosol, catechol, benzoic acid and 4-hydroxybenzaldehyde, respectively.

Table 1

| Compound | Structure | Chemical name |
|---|---|---|
| I | 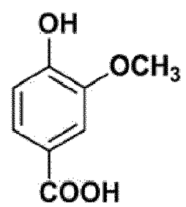 | Vanillic acid |

(continued)

| Compound | Structure | Chemical name |
|---|---|---|
| II | 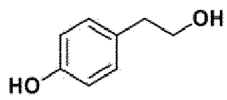 | Tyrosol |
| III | 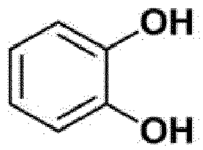 | Catechol |
| IV | 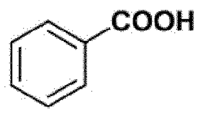 | Banzoic acid |
| V | 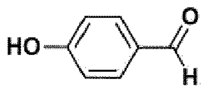 | 4-hydroxybenzaldehyde |

**Example 1: SOD activity of the fermentation product of black rice extract**

It is known that the enhancement of SOD activity is helpful for enhancing the oxidation-inhibitory ability of cells. Therefore, if the SOD activity can be enhanced, the effect of inhibiting skin aging can be achieved. To know the SOD activity of the fermentation product of black rice extract of the present invention, the following test was conducted.

Experimental group: including the "black rice extract group," "black rice fermentation product group," and "white rice fermentation product group," wherein the samples of the aforesaid group were 20 μl of black rice extract provided by [Preparation example A-1], 20 μl of fermentation product of black rice extract provided by [Preparation example A-4], and 20 μl of fermentation product of white rice extract provided by [Preparation example B], respectively, and 2.35 ml of A liquid (0.1 M Tris-HCl and 1 mM EDTA), 1.8 ml of $H_2O$ and 0.15 ml of B liquid (4.5 mM pyrogallol hydrochloride liquid) were sequentially added into the sample of each group.

Blank group: 2.35 ml of A liquid, 1.8 ml of $H_2O$ and 0.15 ml of B liquid were sequentially mixed.

After the B liquid was added, a UV/VIS Spectrophotometer was used to test the A325 absorbance of each group at the time points of 0 second and 1 minute after the reaction was conducted.

The SOD activity each of the "black rice extract group," "black rice fermentation product group," and "white rice fermentation product group" was calculated by using the following arithmetic formula (I). The results are shown in Figures 2 and 3.

$$\text{Arithmetic formula (I): SOD activity (U/mL)}= \frac{\frac{\triangle A325-\triangle A'325}{\triangle 325} X 100\%}{50\%} X\ 4.5\ X\ \frac{1}{V}\ X\ D$$

ΔA325 was the difference between "the A325 absorbance of the blank group at 0 second" and "the A325 absorbance of the blank group at 1 minute after the response was conducted;" ΔA'325 was the difference between "the A325 absorbance of the experimental group (i.e., the "black rice extract group," "black rice fermentation product group," or "white rice fermentation product group") at 0 second" and "the A325 absorbance of the experimental group at 1 minute after the response was conducted;" V was the volume of the sample, wherein its unit was ml; and D was the dilution times of sample.

As shown in Figures 2 and 3, no matter the comparison between the "black rice extract group" or "white rice fermentation product group," the "black rice fermentation product group" has a significantly higher SOD activity. These results indicate that by fermenting a black rice extract with the fermentation method of the present invention, the SOD activity of the product can be effectively enhanced. On the other hand, as compared to the fermentation product provided by using white rice as the material, the product provided by using black rice has a better SOD activity. This illustrates that the fermentation product of black rice extract of the present invention is helpful for enhancing the oxidation-inhibitory ability of cells, and thus can be used for inhibiting skin aging, and its effect(s) is better than that of the fermentation product of white rice extract.

**Example 2: Test for the effect of the fermentation product of black rice extract on inhibiting the production of AGEs**

The materials and equipment used in the following example are described as below:

200 mM sodium phosphate buffer, pH 7.4;
60 mg/ml collagen, which contains 0.06% $NaN_3$ and is prepared with 200 mM sodium phosphate buffer;
1.5 M D-fructose, which is prepared with 200 mM sodium phosphate buffer; and
ELISA reader.

Experimental group:

I. 0.4 ml each of the black rice extract provided by [Preparation example A-1], fermentation product of black rice extract provided by [Preparation example A-4], and fermentation product of white rice extract provided by [Preparation example B] was respectively used as the sample of the "black rice extract group," "black rice fermentation product group," and "white rice fermentation product group," and separately mixed with collagen solution (0.4 ml) and fructose solution (0.4 ml) evenly to provide three mixed solutions;
II. 0.1 ml was taken from each of the mixed solutions provided by step I to be the sample for detecting the fluorescence intensities at an excitation wavelength of 360 nm and an emission wavelength of 460 nm, and the aforesaid fluorescence intensity detection was repeated three times for each group, and then, fluorescence intensities of the three detections were averaged to be the "fluorescence intensity of experimental group at hour 0;"
III. The mixed solutions provided by step I reacted at 50°C for 24 hours, and thus, the collagen contained in the solutions could be glycosylated, so as to provide three glycosylated solutions; and
IV. 0.1 ml was taken from each of the glycosylated solutions provided by step III to be the sample for detecting the fluorescence intensities at an excitation wavelength of 360 nm and an emission wavelength of 460 nm, and the aforesaid fluorescence intensity detection was repeated three times for each group, and then, fluorescence intensities of the three detections were averaged to be the "fluorescence intensity of experimental group at hour 24."

B. Control group:

i. 0.4 ml of water was mixed with collagen solution (0.4 ml) and fructose solution (0.4 ml) evenly to provide a mixed solution;
ii. 0.1 ml of the mixed solution provided by step i was used as the sample for detecting the fluorescence intensities at an excitation wavelength of 360 nm and an emission wavelength of 460 nm, and the fluorescence intensity detection was repeated three times, and then, fluorescence intensities of the three detections were averaged to be the "fluorescence intensity of control group at hour 0;"
iii. The mixed solution provided by step i reacted at 50°C for 24 hours, and thus the collagen contained in the solution could be glycosylated, so as to provide a glycosylated solution; and
iv. 0.1 ml of the glycosylated solution provided by step iii was used as the sample for detecting the fluorescence intensities at an excitation wavelength of 360 nm and an emission wavelength of 460 nm, and the fluorescence intensity detection was repeated three times, and then, fluorescence intensities of the three detection were averaged to be the "fluorescence intensity of control group at hour 24."

Thereafter, the glycosylation-inhibitory activity (%) of each experimental group was calculated by using the following arithmetic formula (II). The results are shown in Figures 4 and 5.

$$\text{Glycosylation-inhibitory activity (\%)} =$$

$$\left[1-\frac{\text{fluorescence intensity of experimental group at hour 24}-\text{fluorescence intensity of experimental group at hour 0}}{\text{fluorescence intensity of control group at hour 24}-\text{fluorescence intensity of control group at hour 0}}\right]x100\%$$

As shown in Figures 4 and 5, no matter the comparison between the "black rice extract group" or "white rice fermentation product group," the "black rice fermentation product group" has a significantly better effect on inhibiting

glycosylation. These results indicate that by fermenting a black rice extract with the fermentation method of the present invention, the glycosylation-inhibitory activity (inhibiting the production of AGEs) of the product can be effectively enhanced. On the other hand, as compared to the fermentation product provided by using white rice as the material, the product provided by using black rice has a better glycosylation-inhibitory activity (inhibiting the production of AGEs). This illustrates that the fermentation product of black rice extract of the present invention can achieve the effects of tightening skin, enhancing skin elasticity, reducing wrinkles and inhibiting skin aging by inhibiting the production of AGEs, and its effect(s) is better than that of the fermentation product of white rice extract.

**Example 3: Test for the content of moisturizing polysaccharide in the fermentation product of black rice extract**

It is known that moisturizing polysaccharide is effective in maintaining the moisture of the skin. To know whether the fermentation product of black rice extract of the present invention has a higher content of moisturizing polysaccharide, the following test for the moisturizing polysaccharide content was conducted.

Plotting standard curve:

I. In a 10 ml container, 10 mg glucose was prepared into a 1 mg/ml glucose solution with $ddH_2O$;
II. The glucose solution provided by step I was serially diluted into 0, 20, 50, 100, 150 and 200 μg/ml glucose solution with $ddH_2O$;
III. The glucose solutions provided by step II were put separately into different glass tubes (100 μl solution for each tube);
IV. 500 μl of 5% phenol solution and 2.5 ml of sulfuric acid solution (95.5%) were added into each of the above glass tubes, which were then vortex mixed and then stood for 20 minutes, so as to provide six mixed solutions;
V. The mixed solutions of each group provided by step IV were put separately into different wells of a 96-well plate (200 μl solution for each well), and the absorbance at 490 nm of each solution was detected for plotting standard curve.

Experimental group:

i. 100 μl each of black rice extract provided by [Preparation example A-1], fermentation product of black rice extract provided by [Preparation example A-4], and fermentation product of white rice extract provided by [Preparation example B] was respectively used as the sample of the "black rice extract group," "black rice fermentation product group," and "white rice fermentation product group," and put separately into glass tubes;
ii. 500 μl of 5% phenol solution and 2.5 ml of sulfuric acid solution (95.5%) were added into each of the above glass tubes, which were then vortex mixed and then stood for 20 minutes, so as to provide three mixed solutions;
iii. The mixed solutions of each group provided by step ii were put separately into a different well of a 96-well plate (200 μl for each well), and the absorbance at 490 nm of each solution was detected by a ELISA reader, and then, the moisturizing polysaccharide content each of the black rice extract provided by [Preparation example A-1], fermentation product of black rice extract provided by [Preparation example A-4], and fermentation product of white rice extract provided by [Preparation example B] was calculated by using interpolation method, and the results are shown in Figures 6 and 7.

As shown in Figures 6 and 7, no matter the comparison between the "black rice extract group" or "white rice fermentation product group," the "black rice fermentation product group" has a significantly higher content of moisturizing polysaccharide. These results indicate that by fermenting a black rice extract with the fermentation method of the present invention, the moisturizing polysaccharide content of the product can be effectively enhanced. On the other hand, as compared to the fermentation product provided by using white rice as the material, the product provided by using black rice has a higher content of moisturizing polysaccharide and thus is helpful for enhancing the moisturizing ability of the skin. This illustrates that the fermentation product of black rice extract of the present invention can be used for moisturizing skin, and its effect(s) is better than that of the fermentation product of white rice extract.

**Example 4: Test for the effect of the fermentation product of black rice extract on enhancing expression of aquaporin in skin**

Aquaporin is the channel located on the cell membrane that is responsible for the active transportation of water.

As compared to normal water diffusion, aquaporin can supply the moisture content to skin cells faster. To know whether the fermentation product of black rice extract of the present invention can enhance the expression of aquaporin in skin cells, the following aquaporin fluorescent dying test was conducted.

The materials and equipment used in the following example are described as below:

1. Cell line: human primary epidermal keratinocytes HPEK-50, purchased from CELLnTEC;
2. Medium: Keratinocyte-SFM (IX), purchased from Thermo;
3. Primary antibody: Polyclonal anti-AQP3 antibody, purchased from Boster immunoleader;
4. Secondary antibody: Anti-mouse-alexa 488 antibody, purchased from Thermo;
5. Hoechst dye: Hoechst 33342, purchased from Thermo;
6. Triton X-100: purchased from Sigma;
7. BSA: purchased from Bio Basic Inc; and
8. 10% formaldehyde purchased from ECHO.

HPEK-50 cells were separated into three groups ($2x10^3$ cells/group) and inoculated into different cell chamber slides, and then, the three chamber slides were respectively placed into the following medium for culturing over night:

1. Control group: a medium free of the black rice extract and the fermentation product of black rice extract;
2. Black rice extract group: a medium containing 4% black rice extract provided by [Preparation example A-1];
3. Black rice fermentation product group: a medium containing 4% fermentation product of black rice extract provided by [Preparation example A-4].

The cells provided by the above groups were subjected to the following treatments: fixing the cells with 4% trioxane for 15 minutes at room temperature; washing the cells with 1xPBS for three times, and then, adding 0.5% Triton X-100 and keeping the cells at room temperature for 10 minutes to penetrate the cells; adding 1%BSA and keeping at room temperature for 1 hour to block the reaction; washing the cells with 1xPBS for three times, and then, adding primary anti-body (diluted with 1% BSA) and culturing at 37°C for 2 hours; washing the cells with 1xPBS for three times, and then, adding secondary anti-body (diluted with 1% BSA) and culturing at 37°C for 1 hour; washing the cells with 1xPBS for three times, and then, adding Hoechst dye and culturing at room temperature for 3 to 5 minutes; washing the cells with 1xPBS for three times, and then, mounting the cells with mounting medium, and observing and photographing the slides under a fluorescence microscope. The results are shown in Figure 8 (the portion with blue fluorescence was nucleus; the portion with green fluorescence was aquaporin).

As shown in Figure 8, no matter the comparison between the "control group" or "black rice extract group," the "black rice fermentation product group" has a significantly higher expression of aquaporin. These results indicate that, the fermentation product of black rice extract of the present invention is effective in enhancing expression of aquaporin, and thus, is helpful for enhancing the moisturizing ability of the skin. This illustrates again that the fermentation product of black rice extract of the present invention can be used for moisturizing the skin.

**Example 5: Test for the effect of fermentation product of black rice extract on tightening skin**

A cell experiment that mimics the growth of skin tissue was conducted in this example, wherein, if an analyte makes the skin tissue grow more tightly (i.e., making the area of formed tissue to be smaller, and increasing the density of collagen), the analyte is more effective in tightening the skin.

The materials and equipment used in the following example are described as below:

1. Cell line: Human skin fibroblast, CCD-966sk (BCRC No. 60153);
2. Medium: 90% Minimum essential medium (Eagle) in Earle's BSS containing 0.1 mM non-essential amino acids, 1.5g/L sodium bicarbonate and 1 mM sodium pyruvate, and 10% fetal bovine serum; purchased from Gibco;
3. PBS: purchased from Gibco;
4. Collagen I: purchased from Gibco; and
5. NaOH.

CCD-966sk cells were separated into three groups ($2x10^5$ cells/group) and added into different sterilized tubes. 3 mg/ml collagen solution was added into each tube to provide three mixed solutions (the volume ratio of cell suspension and collagen solution in each group was 0.66: 0.33 (cell suspension: collagen solution)). Thereafter, a little 1M NaOH (in an amount that can change the phenol red media indicator into light pink color) was quickly added into each mixed solution, and then the mixed solution was pipetted up and down to mix. The evenly mixed solution of each group (500 μl for each solution) was put into the well of a 24-well plate (1.9 $cm^2$/ well), and the wells were covered and the plate

was placed at room temperature for 20 minutes, so as to provide collagen gels.

500 μl each of the following media was respectively added into the aforementioned wells of different group:

1. Control group: medium free of the black rice extract and the fermentation product of black rice extract;
2. Black rice extract group: medium containing 4% black rice extract provided by [Preparation example A-1];
3. Black rice fermentation product group: medium containing 4% fermentation product of black rice extract provided by [Preparation example A-4].

Thereafter, the collagen gels were gently detached from the wells by using the tip of a 200 μl pipette and resuspended. The 24-well plate was cultured at 37°C and 5% humidified $CO_2$ for 48 hours. Lastly, the 24-well plate was put on a light box and photographed with a digital camara from the top of the 24-well plate. The outline of each collagen gel was traced by using Image J software, and the area of each collagen gel was calculated. The photos are shown in Figure 9 (the yellow line was the outline of the collagen gel obtained from the "control group," the blue line was the outline of the collagen gel obtained from the "black rice extract group," and the red line was the outline of the collagen gel obtained from the "black rice fermentation product group"). Lastly, the relative collagen density of each group was calculated by using the result of the control group as a basis (i.e., set the collagen density of the control group as 100%). Data were calculated and analyzed with Excel software, and student's t-test was used for determining whether the data has statistical significance. The results are shown in Figure 10.

As shown in Figures 9 and 10, no matter the comparison between the "control group" or "black rice extract group," the "black rice fermentation product group" has a smaller area of collagen gel and a higher collagen density. These results indicate that the fermentation product of black rice extract of the present invention is effective in tightening skin.

**Example 6: Human trials**

**(6-1) Short-term trial of applying mask that contains fermentation product of black rice extract**

The experimentation was carried out in a self-control way by five subjects (healthy men/women between 25 to 55 years old). The arm of each subject was delineated into two parts, and the two parts were respectively applied with black rice fermentation product mask (containing 1% fermentation product of black rice extract provided by [Preparation example A-4], based on the total weight of the essence in mask) and a placebo mask (free of the fermentation product of black rice extract of the present invention, but other ingredients were all the same as the black rice fermentation product mask). After applying the masks for 20 minutes, the masks were taken off, and the skin was massaged gently with finger pulps to enhance the absorption of the essence in the masks. The skin moisture content, skin sagging, and skin elasticity were detected and recorded by using a DermaLab® Combo Skin Analyzer at 0 minute (i.e., prior to starting applying the mask) and $20^{th}$ minute (i.e., after applying the mask for 20 minutes), respectively. Then, the result of 0 minute was used as a basis (i.e., the result of 0 minute were set as 100%) to calculate the skin moisture content, skin sagging and skin elasticity after applying the mask for 20 minutes. The results are shown in Figures 11 to 13.

As shown in Figures 11 to 13, as compared to applying the placebo mask, after applying the black rice fermentation product mask for 20 minutes, the skin moisture content and skin elasticity of the subjects significantly increased, and the skin sagging of the subjects significantly decreased. These results indicate that fermentation product of black rice extract of the present invention is effective in enhancing skin moisture content and skin elasticity as well as tightening skin in a brief period of time.

**(6-2) Long-term trial of eating fermentation product of black rice extract**

This experimentation was carried out in a self-control way by nigh subjects (men/women between 25 to 60 years old having skin sagging). Each subject drunk a bottle (5 ml) of black rice fermentation product beverage (containing 10% fermentation product of black rice extract provided by [Preparation example A-4], based on the total weight of the beverage) for 4 weeks. The skin of those subjects was detected and recorded by using a DermaLab® Combo Skin Analyzer, and a VISIA® Skin Analysis System at week 0 (i.e., prior to starting drinking the beverage containing the fermentation product of black rice extract of the present invention) and week 4 (i.e., after drinking the beverage containing the fermentation product of black rice extract of the present invention for 4 weeks), respectively. Then, the data thus obtained was analyzed by Student t-test, and the result of week 0 was used as a basis (i.e., the result of week 0 was set as 100%) to calculate the skin moisture content, skin elasticity, skin sagging and skin wrinkles after drinking the beverage containing the fermentation product of black rice extract of the present invention for 4 weeks. The results of skin moisture content, skin elasticity, skin sagging, and skin wrinkles are shown in Figures 14 to 17. And, Figure 18 shows the photos of skin wrinkles of two of the subjects.

As shown in Figures 14 to 18, after drinking the beverage containing the fermentation product of black rice extract of the present invention for 4 weeks, the skin moisture content, and skin elasticity of the subjects significantly increased, and the skin sagging and skin wrinkles of the subjects significantly decreased. These results indicate that the fermentation product of black rice extract of the present invention is indeed effective in moisturizing skin, enhancing skin elasticity, tightening skin and reducing skin wrinkles.

**Example 7: Test for the effect of compounds of the present invention on promoting secretion of collagen**

The materials and equipment used in the following example are described as below:

1. Cell line: Human skin fibroblast, CCD-966Sk, purchased from ATCC®, product number: CRL-1881;
2. Medium: MEM medium containing 10% FBS, 1% penicillin/streptomycin and 1 mM sodium pyruvate, purchased from Gibco;
3. PBS: purchased from Gibco;
4. Sircol™ Soluble Collagen Assay kit: purchased from Biocolor; and
5. ELISA reader: purchased from BioTek.

CCD-966Sk cells were separated into six groups ($2x10^4$ cells/group) and inoculated into different wells of a 24-well plate (500 μl of medium in each well). After culturing for 24 hours at 37°C, the cells were washed with PBS. The cells of each group were then cultured in the following medium for 48 hours:

1. Control group: medium free of the compounds of formula (I) to (V) (500 μl in total);
2. Group I: medium containing 20 μg/ml compound of formula (I) provided by [Preparation example C] (500 μl in total);
3. Group II: medium containing 20 μg/ml compound of formula (II) provided by [Preparation example C] (500 μl in total);
4. Group III: medium containing 20 μg/ml compound of formula (III) provided by [Preparation example C] (500 μl in total);
5. Group IV: medium containing 20 μg/ml compound of formula (IV) provided by [Preparation example C] (500 μl in total); and
6. Group V: medium containing 20 μg/ml compound of formula (V) provided by [Preparation example C] (500 μl in total).

Thereafter, the cells provided by the above groups were subjected to the following treatments: respectively transferring the medium of each group into 1.5 ml centrifugation tube; adding 200 μl Collagen Isolation & Concentration Reagent into each tube, these tubes were then put upside down 6 to 8 times, and these tubes then stood at 4°C overnight; thereafter, centrifuging these tubes for 10 minutes at 12000 rpm, and removing the supernatant; adding 1000 μl Sircol dye into each tube, and gently shaking the tubes for 30 minutes; centrifuging these tubes for 10 minutes at 12000 rpm again, and removing the supernatant; thereafter, adding 750 μl acid-salt washing reagent into each tube, and centrifuging these tubes for 10 minutes at 12000 rpm; removing supernatant, and then putting the tubes upside down to empty the liquid from the tube, and removing the remnant liquid with cotton swab; lastly, adding 250 μl alkali reagent into each tube and mixing evenly, and then reading the absorbance of the liquid thus obtained at 555 nm by an ELISA reader; analyzing the obtained data by Student t-test, and the result of the "control group" was used as a basis (i.e., the collagen secretion level of the control group was set as 100%) to calculate the relative collagen secretion level of the "group I" to "group V." The results are shown in Figure 19.

As shown in Figure 19, as compare to the "control group," the collagen secretion of the cells provided by the "group I" to "group III" and "group V" were significantly increased. These results indicate that compounds of formula (I) to (III) and (V) are effectively in enhancing skin elasticity, tightening skin and reducing wrinkles.

**Example 8: Test for the effect of compounds of the present invention on promoting expression of elastin**

The materials and equipment used in the following example are described as below:

1. Cell line: Human skin fibroblast, CCD-966Sk, purchased from ATCC®, product number: CRL-1881;
2. Medium: MEM medium containing 10% FBS, 1% penicillin/streptomycin and 1 mM sodium pyruvate, purchased from Gibco;
3. PBS : purchased from Gibco;
4. Fastin™ Elastin Assay kit: purchased from Biocolor; and
5. ELISA reader: purchased from BioTek.

CCD-966Sk cells were separated into six groups (1x$10^5$ cells/group) and inoculated into different well of a 6-well plate, and then the plate was placed at 37°C 24 hours. Thereafter, the cells were cultured in the following medium for 48 hours:

1. Control group: medium free of the compounds of formula (I) to (V) (2 ml in total);
2. Group I: medium containing 20 μg/ml compound of formula (I) provided by [Preparation example C] (2 ml in total);
3. Group II: medium containing 20 μg/ml compound of formula (II) provided by [Preparation example C] (2 ml in total);
4. Group III: medium containing 20 μg/ml compound of formula (III) provided by [Preparation example C] (2 ml in total);
5. Group IV: medium containing 20 μg/ml compound of formula (IV) provided by [Preparation example C] (2 ml in total); and
6. Group V: medium containing 20 μg/ml compound of formula (V) provided by [Preparation example C] (2 ml in total).

Thereafter, the cells provided by the above groups were subjected to the following treatments: removing medium of each group, and washing the cells with 1xPBS once; treating cells with trypsin for 3 minutes; thereafter, blocking the activity of trypsin with medium; transferring the trypsin treated cells into 1.5 ml microcentrifuge tubes, and centrifuging the tubes for 5 minutes at 300xg; washing the cells with PBS, and then centrifuging the tubes for 5 minutes at 300xg again; thereafter, reserving the cell pellets in 300 μl of PBS, and adding 100 μl 1.0M oxalic acid therein, and then culturing the tubes for 1 hour at 100°C; adding equivalent Elastin Precipitating Reagent into each tube and mixing evenly, and then standing the tubes for 15 minutes; centrifuging the tubes for 10 minutes at 10,000xg, and emptying the liquid from the tubes; thereafter, adding 1 ml dye into each tube and mixing evenly, and then culturing for 90 minutes on a shaking incubator; after emptying the dye that did not bind to the elastin, the elastin in a reddish brown color that precipitated in the bottom of the tubes can be seen; adding 250 μl Dye Dissociation Reagent into each tube, and releasing the dye into the solutions by vortex mixer; transferring the solutions of each group into different wells of a 96-well plate, and reading the absorbance at 513 nm of the solutions by an ELISA reader; lastly, analyzing the obtained data by Student t-test, and the result of the "control group" was used as a basis (i.e., the elastin expression level of the control group was set as 100%) to calculate the relative elastin expression level of the "group I" to "group V." The results are shown in Figure 20.

As shown in Figure 20, as compared to the "control group," the elastin expression of the cells provided by the "group I" to "group V" were significantly increased. These results indicate that compounds of formula (I) to (V) are effectively in enhancing skin elasticity, tightening the skin and reducing wrinkles.

**Example 9: Test for the change of contents of the compounds of the present invention in the black rice extract before and after conducting the fermentation process**

The black rice extract provided by [Preparation example A-1] and the fermentation product of black rice extract provided by [Preparation example A-4] were respectively formulated into a sample solution that had a concentration of 20 mg/ml. Thereafter, 10 μl of each sample solution was used to analyze the ingredients contained therein by HPLC. The analyzing column used in the HPLC was Octadecylsilyl, Mightysil RP-18 GP 250 (250x10 mm, 5 μm), the detecting wavelength was 280 nm, the elution rate was 1.0 ml/min, and the composition of the eluant is shown in the following Table 2:

Table 2:

| Time (min) | Mobile phase A (%) | Mobile phase B (%) |
|---|---|---|
| 0 | 98 | 2 |
| 10 | 98 | 2 |
| 40 | 30 | 70 |
| 50 | 0 | 100 |
| 60 | 0 | 100 |
| 62 | 98 | 2 |
| 70 | 98 | 2 |
| *A: water +0.1% formic acid; B: methanol +0.1% formic acid | | |

The HPLC fingerprints obtained by conducting the above steps are shown in Figure 21, wherein the top figure shows the HPLC fingerprint of the fermentation product of black rice extract, and the bottom figure shows the HPLC fingerprint of the black rice extract. As shown in Figure 21, as compared to the black rice extract, the fermentation product

of black rice extract prepared by the fermentation method of the present invention contains compounds of formula (I) to (III) that are not present in the black rice extract, and have higher contents of compounds of formula (IV) and (V). These results indicate that the method of the present invention for manufacturing the fermentation product of black rice extract can effectively increase the contents of the active ingredients contained in the black rice extract.

As shown in the above Examples, the fermentation product of black rice extract of the present invention and the compounds of formula (I) to formula (V) are effective in inhibiting production of AGEs, inhibiting oxidation, enhancing expression of aquaporin, tightening the skin, enhancing secretion of collagen, and enhancing expression of elastin, and thus can be used for inhibiting skin aging, moisturizing the skin, reducing wrinkles, enhancing skin elasticity and tightening the skin. Additionally, the method of the present invention for manufacturing the fermentation product of black rice extract can effectively increase the contents of the active ingredients contained in the black rice extract.

### Claims

1. A method for manufacturing a fermentation product of black rice extract, **characterized in that** the method comprises the following steps:

   (a) extracting black rice by using a polar solvent to provide a black rice extract;
   (b) fermenting the extract by using *Saccharomyces cerevisiae* to provide an intermediate fermentation product; and
   (c) fermenting the intermediate fermentation product by using *Lactobacillus plantarum* to provide a fermentation product of black rice extract.

2. The method as claimed in claim 1, **characterized in that** the polar solvent is water, alcohol, or a combination thereof.

3. The method as claimed in claim 1 or 2, **characterized in that** an amylase is used in step (a).

4. The method as claimed in any one of claims 1 to 3, **characterized in that** the *Saccharomyces cerevisiae* is *Saccharomyces cerevisiae* BCRC 20271.

5. The method as claimed in any one of claims 1 to 4, **characterized in that** the *Lactobacillus plantarum* is *Lactobacillus plantarum* BCRC 910805.

6. A composition **characterized in that** the composition comprises a fermentation product of black rice extract and the fermentation product of black rice extract is provided by the method as claimed in any one of claims 1 to 5.

7. The composition as claimed in claim 6, **characterized in that** the fermentation product of black rice extract comprises at least one of the following compounds of formula (I) to formula (V):

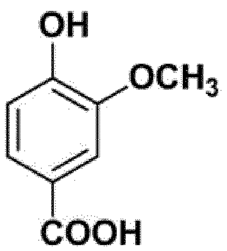 (I), 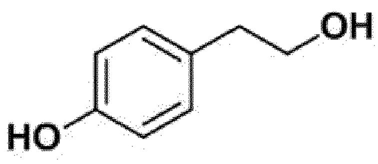 (II), 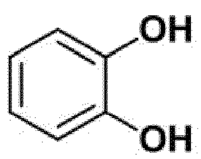 (III),

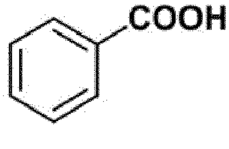 (IV) and 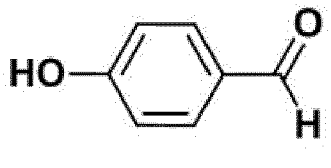 (V).

8. The composition as claimed in claim 6 or 7, **characterized in that** the composition is a pharmaceutical composition, a food composition or a care product composition.

**9.** The composition as claimed in claim 8, **characterized in that** the food composition is a health food, a nutritional supplement or a special nutritional food.

**10.** The composition as claimed in claim 8, **characterized in that** the pharmaceutical composition is formulated for oral administration, injection or transdermal administration.

**11.** A composition for use in inhibiting skin aging, moisturizing skin, reducing wrinkles, enhancing skin elasticity and/or tightening skin, **characterized in that** the composition comprises an effective amount of an active ingredient, and the active ingredient is at least one of the fermentation product of black rice extract provided by the method as claimed in any one of claims 1 to 5 and the following compounds of formula (I) to formula (V):

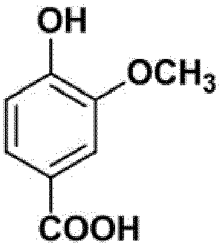
(I),
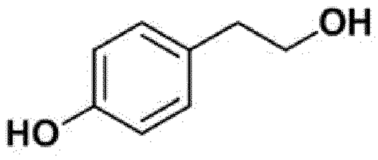
(II),
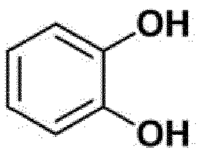
(III),
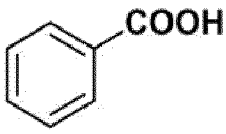
(IV) and
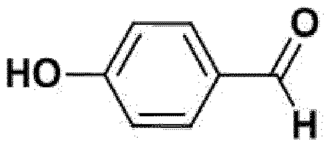
(V).

**12.** The composition for use as claimed in claim 11, **characterized in that** the composition is a food composition or a care product composition.

**13.** The composition for use as claimed in claim 12, **characterized in that** the food composition is a health food, a nutritional supplement or a special nutritional food.

**14.** A pharmaceutical composition for use in at least one of inhibiting production of AGEs, inhibiting oxidation, enhancing expression of aquaporin, enhancing secretion of collagen and enhancing expression of elastin, **characterized in that** the composition comprises an effective amount of an active ingredient, and the active ingredient is at least one of the fermentation product of black rice extract provided by the method as claimed in any one of claims 1 to 5 and the following compounds of formula (I) to formula (V):

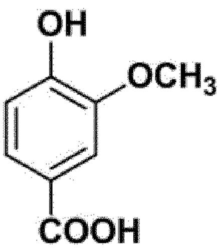
(I),
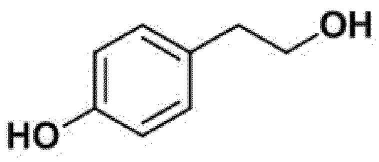
(II),
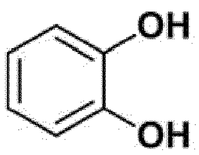
(III),
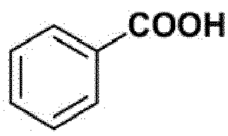
(IV) and
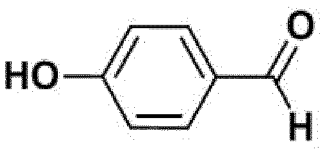
(V).

**15.** The pharmaceutical composition for use as claimed in claim 14, which is formulated for oral administration, injection

or transdermal administration.

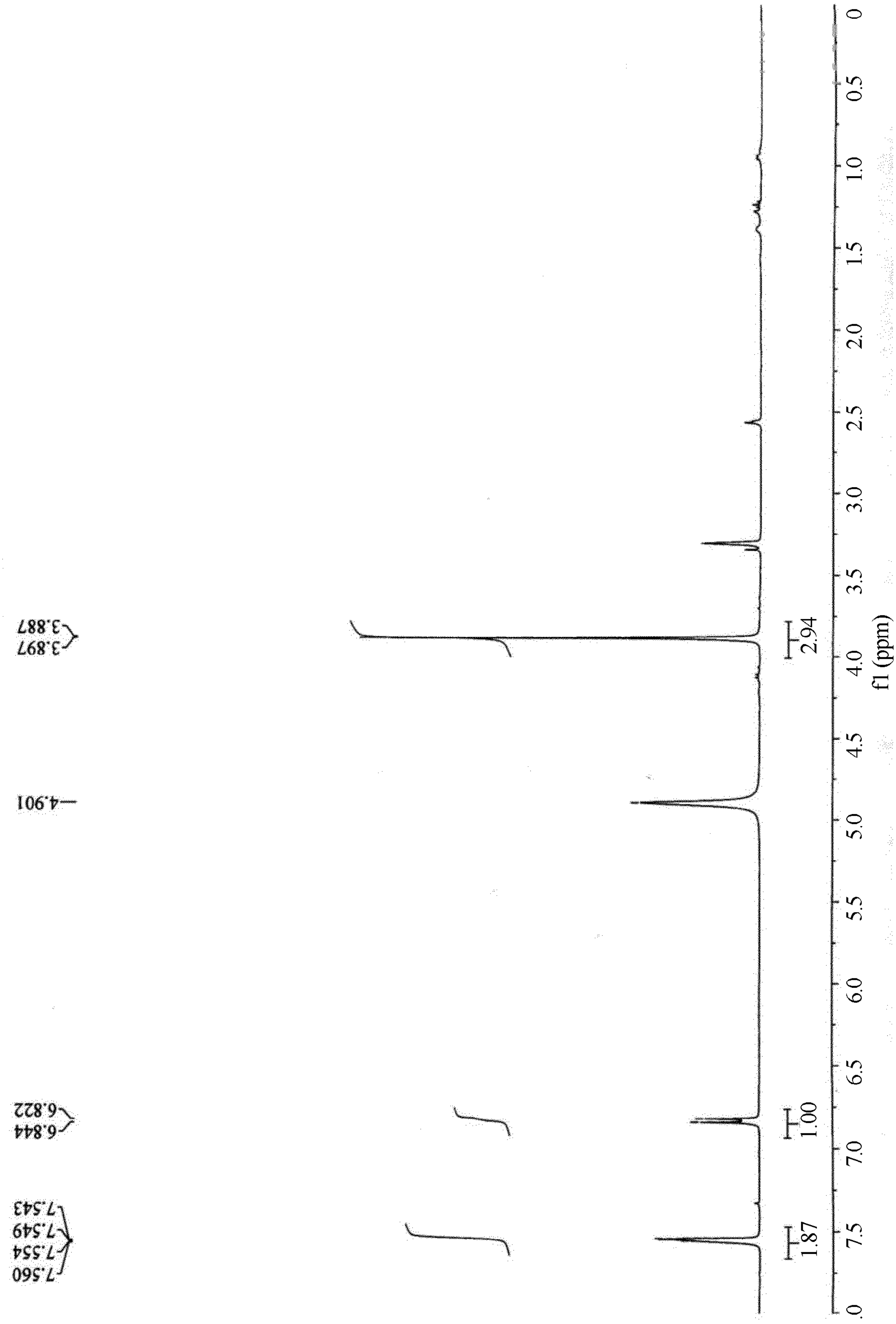
7.560
7.554
7.549
7.543
6.844
6.822
4.901
3.897
3.887
1.87
1.00
2.94
0
0.5
1.0
1.5
2.0
2.5
3.0
3.5
4.0
4.5
5.0
5.5
6.0
6.5
7.0
7.5
f1 (ppm)

FIG. 1A

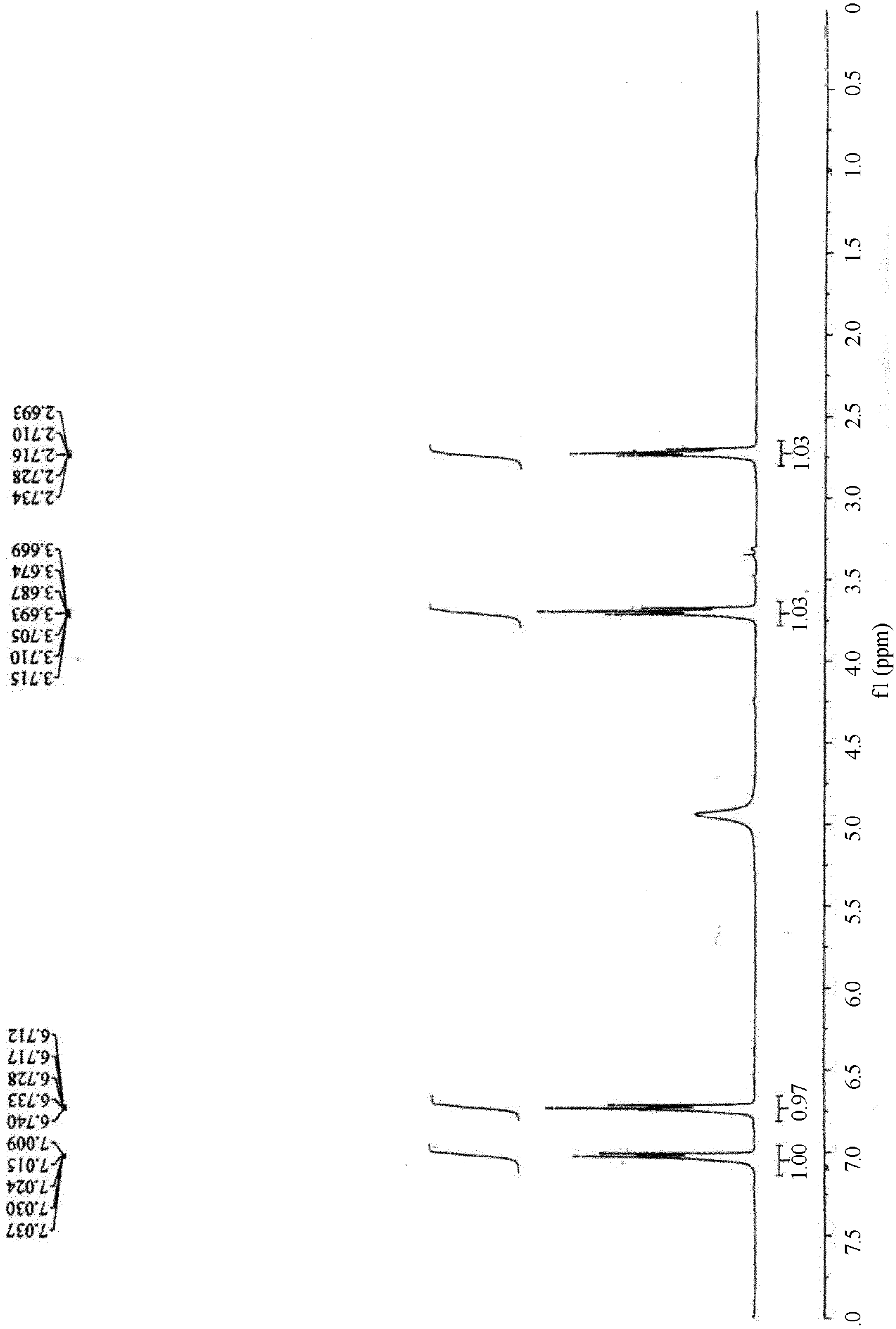
2.693
2.710
2.716
2.728
2.734
3.669
3.674
3.687
3.693
3.705
3.710
3.715
6.712
6.717
6.728
6.733
6.740
7.009
7.015
7.024
7.030
7.037
1.03
1.03
0.97
1.00
0
0.5
1.0
1.5
2.0
2.5
3.0
3.5
4.0
4.5
5.0
5.5
6.0
6.5
7.0
7.5
8.0
f1 (ppm)

FIG. 1B

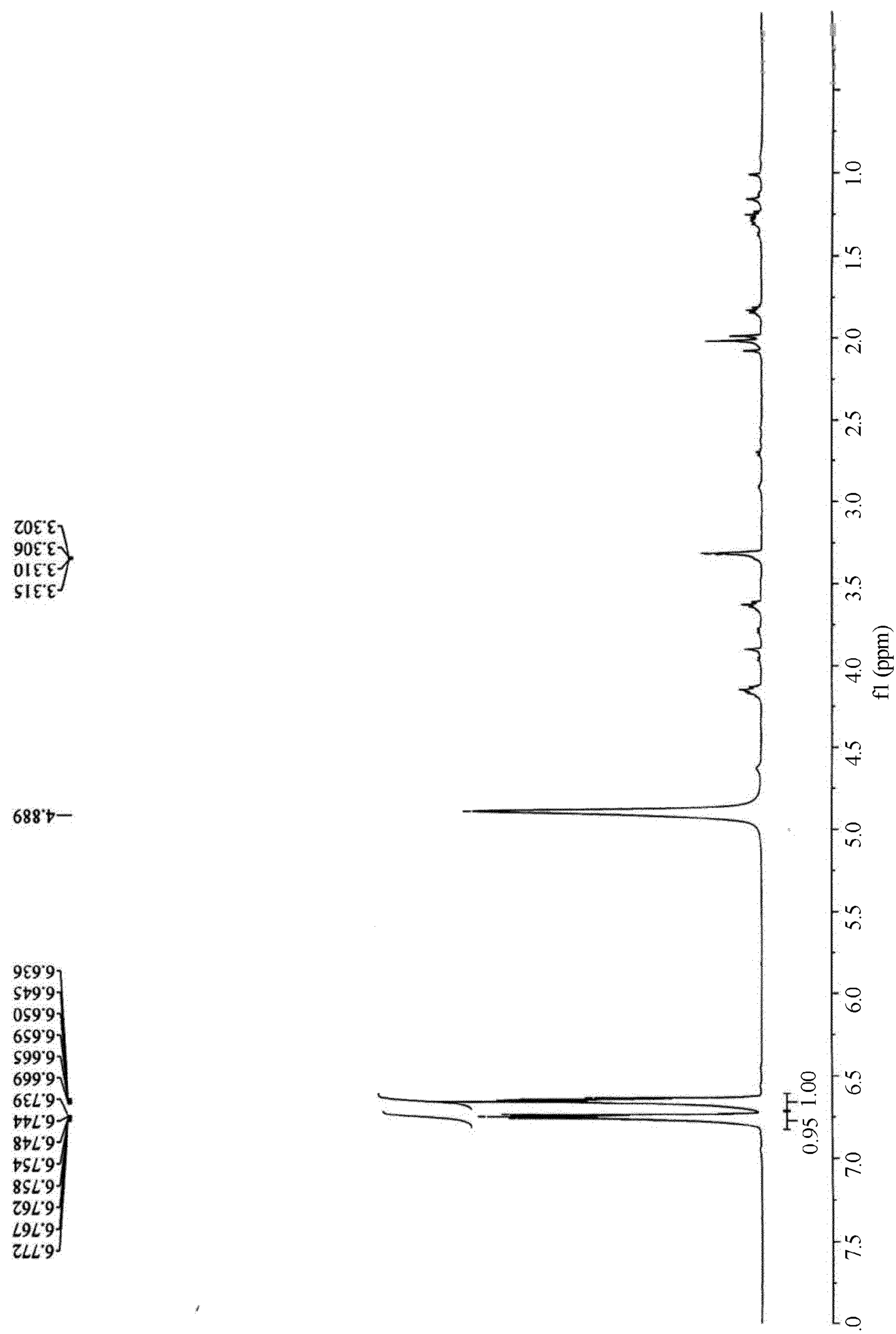
6.772
6.767
6.762
6.758
6.754
6.748
6.744
6.739
6.669
6.665
6.659
6.650
6.645
6.636
4.889
3.315
3.310
3.306
3.302
0.95
1.00
8.0
7.5
7.0
6.5
6.0
5.5
5.0
4.5
4.0
3.5
3.0
2.5
2.0
1.5
1.0
f1 (ppm)

FIG. 1C

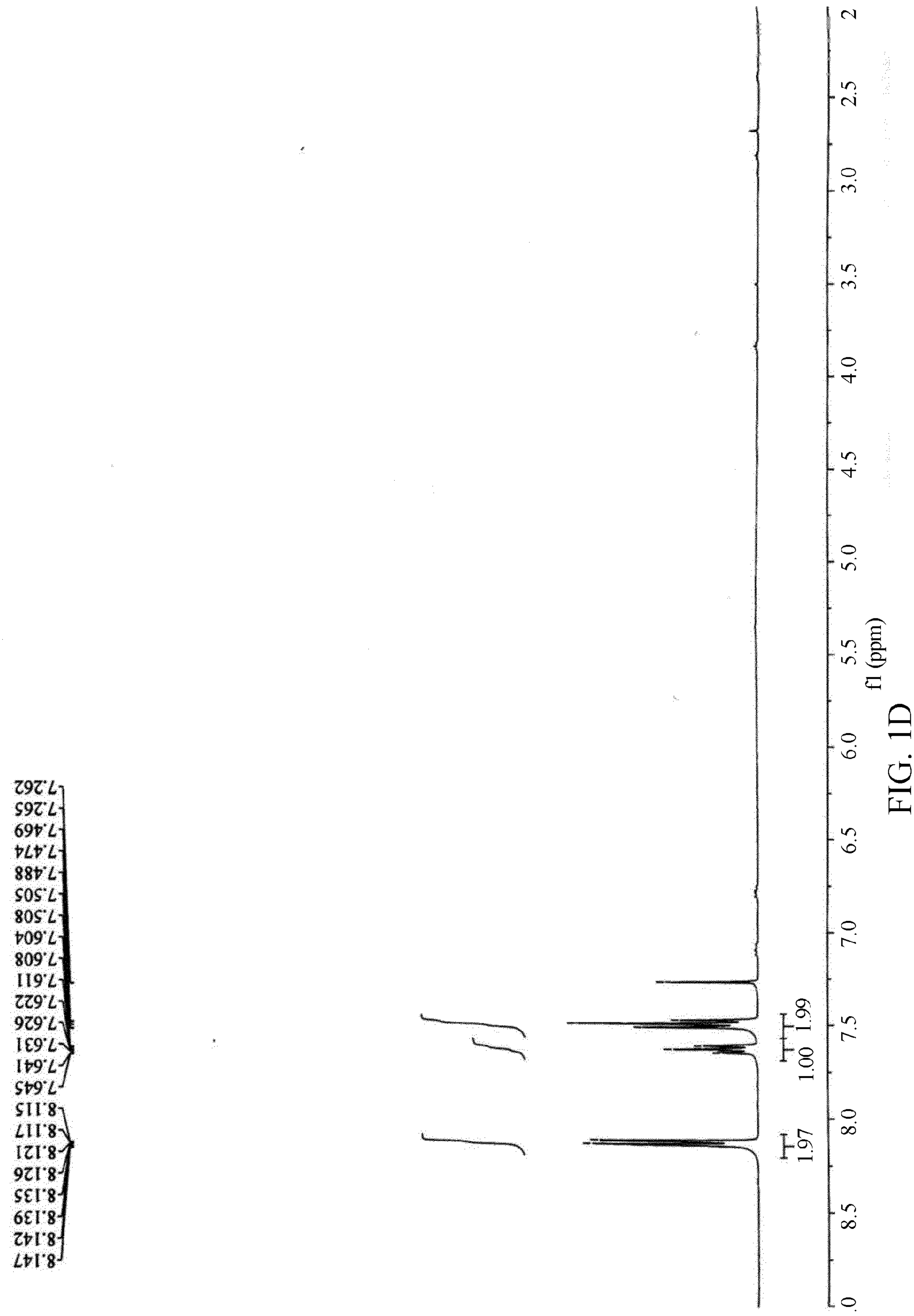
7.262
7.265
7.469
7.474
7.488
7.505
7.508
7.604
7.608
7.611
7.622
7.626
7.631
7.641
7.645
8.115
8.117
8.121
8.126
8.135
8.139
8.142
8.147
1.97
1.00
1.99
2
2.5
3.0
3.5
4.0
4.5
5.0
5.5
6.0
6.5
7.0
7.5
8.0
8.5
f1 (ppm)

FIG. 1D

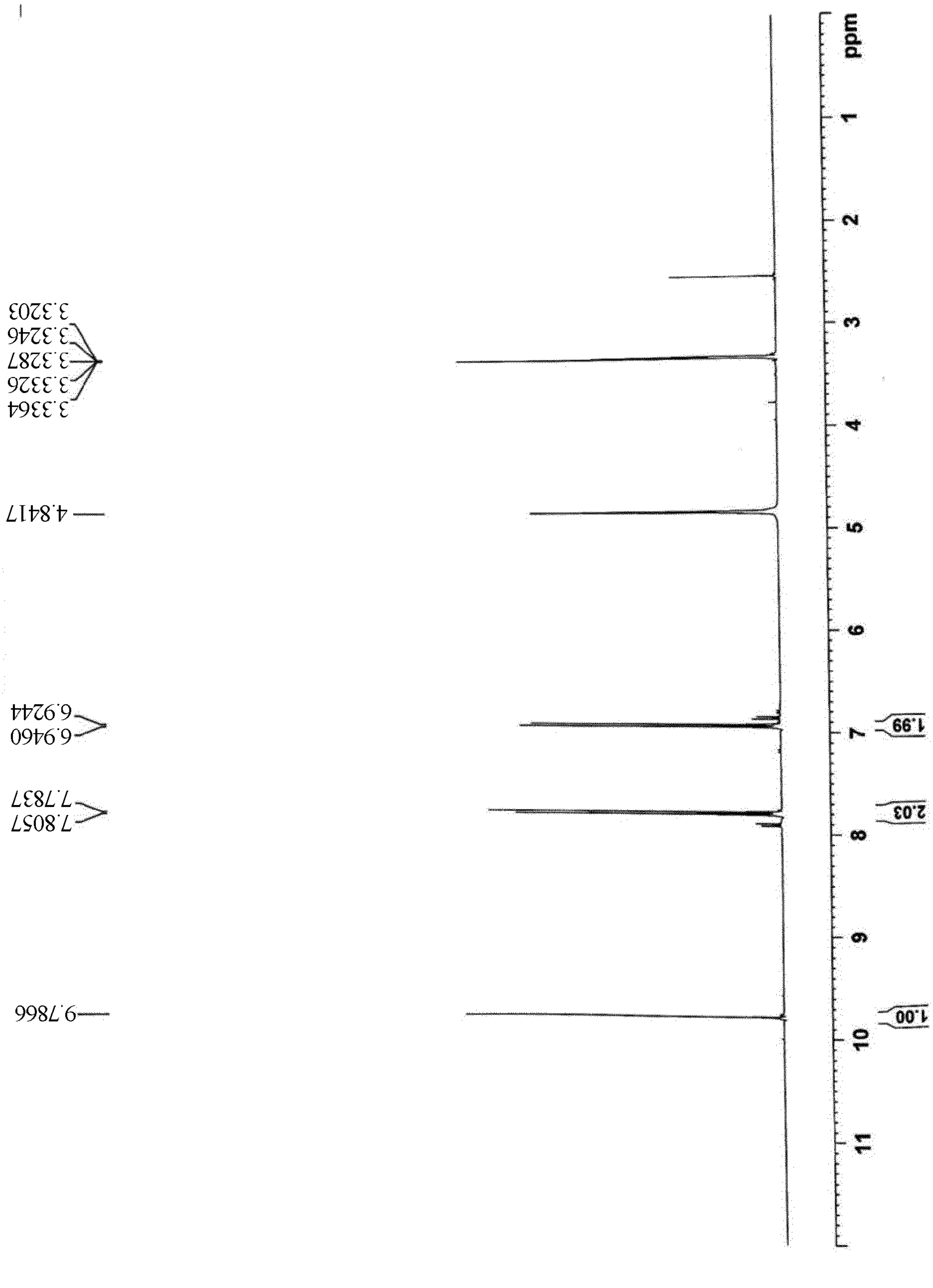
3.3203
3.3246
3.3287
3.3326
3.3364
4.8417
6.9244
6.9460
7.7837
7.8057
9.7866
1.99
2.03
1.00
ppm
1
2
3
4
5
6
7
8
9
10
11

FIG. 1E

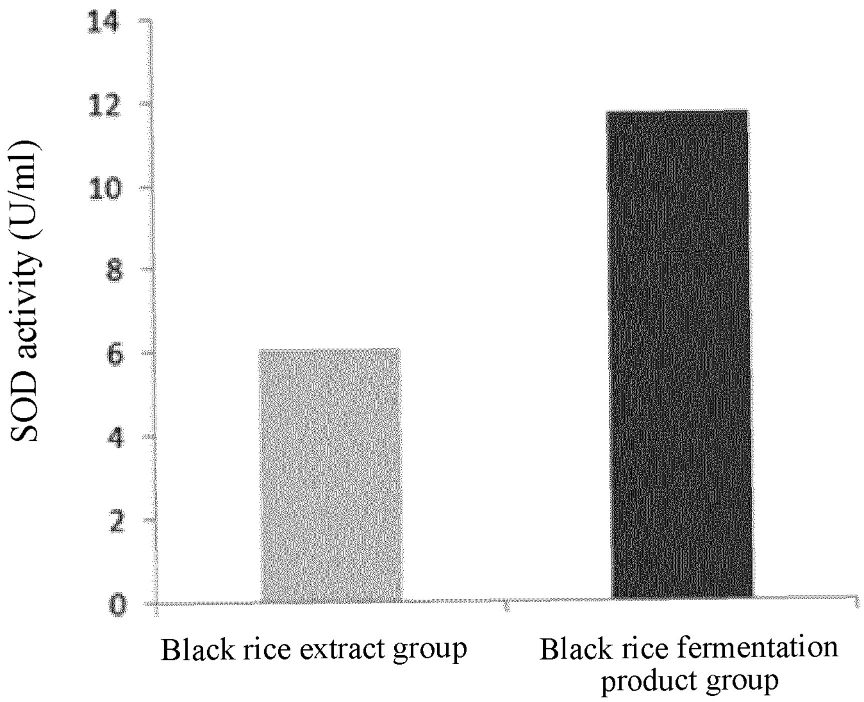
14
12
10
8
6
4
2
0
SOD activity (U/ml)
Black rice extract group
Black rice fermentation product group

FIG. 2

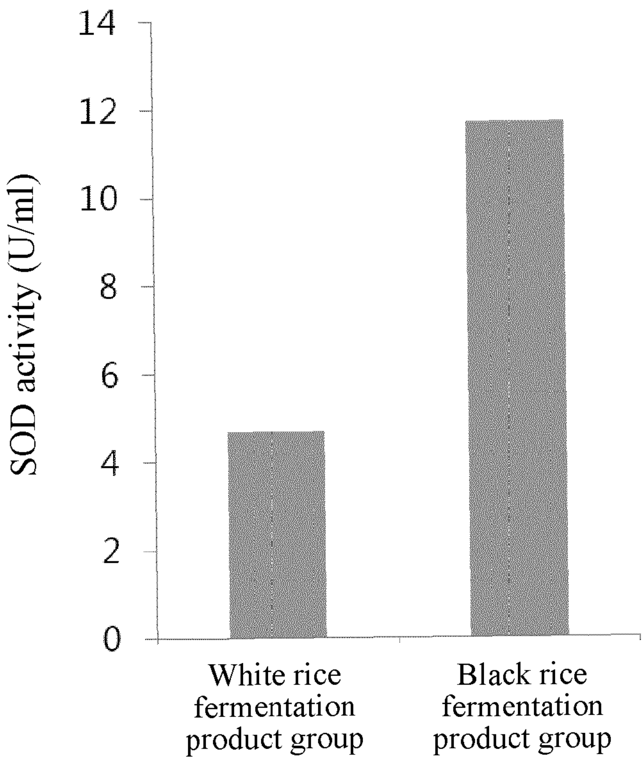
14
12
10
8
6
4
2
0
SOD activity (U/ml)
White rice fermentation product group
Black rice fermentation product group

FIG. 3

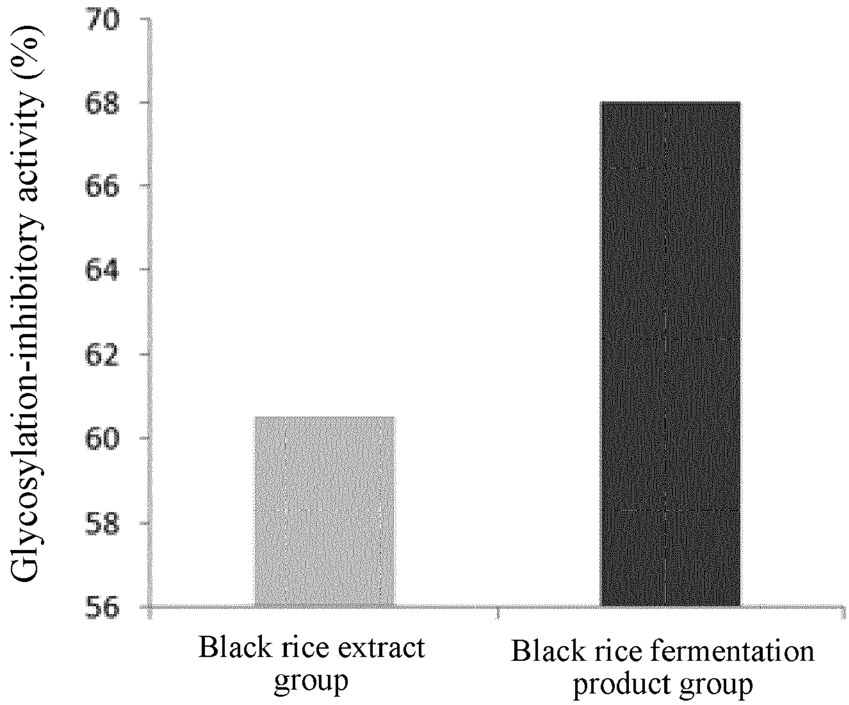
Glycosylation-inhibitory activity (%)
70
68
66
64
62
60
58
56
Black rice extract group
Black rice fermentation product group

FIG. 4

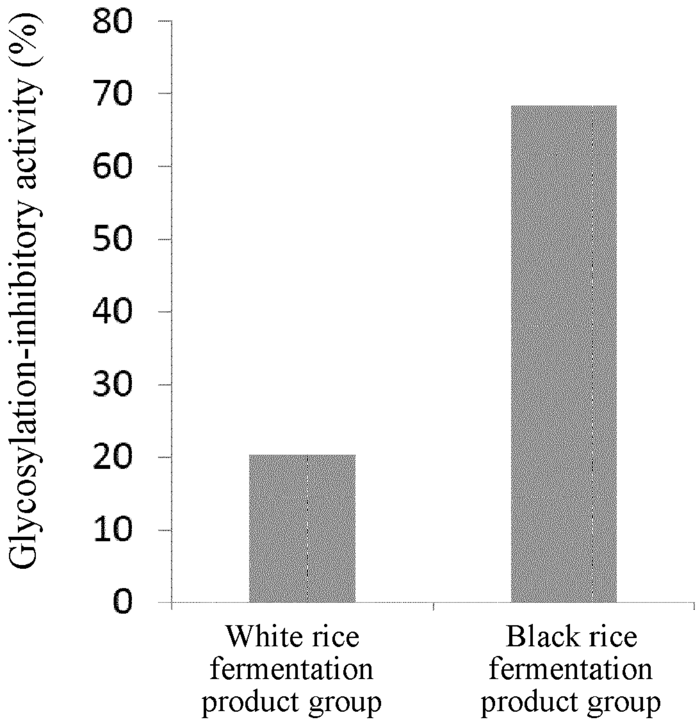
Glycosylation-inhibitory activity (%)
80
70
60
50
40
30
20
10
0
White rice fermentation product group
Black rice fermentation product group

FIG. 5

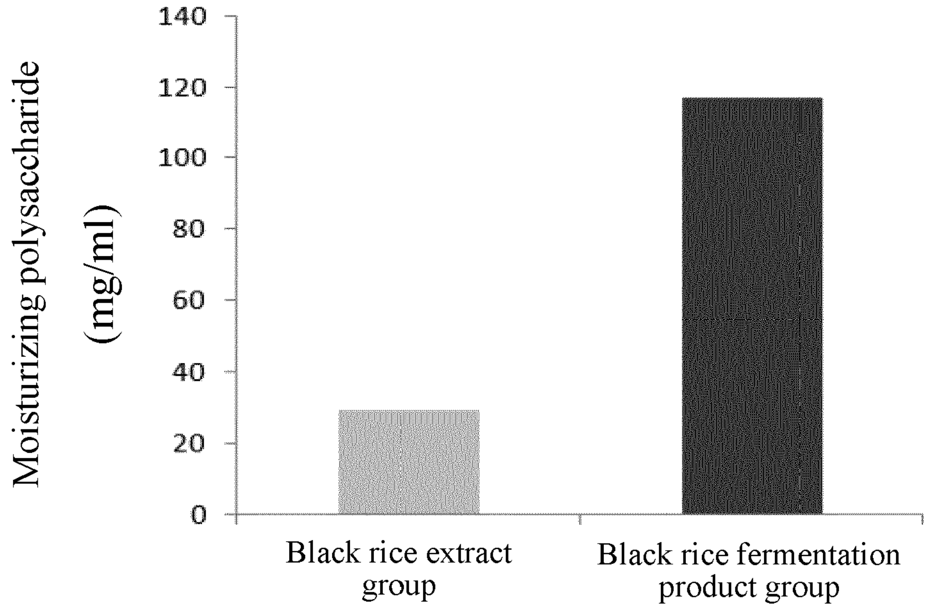
Moisturizing polysaccharide (mg/ml)
140
120
100
80
60
40
20
0
Black rice extract group
Black rice fermentation product group

FIG. 6

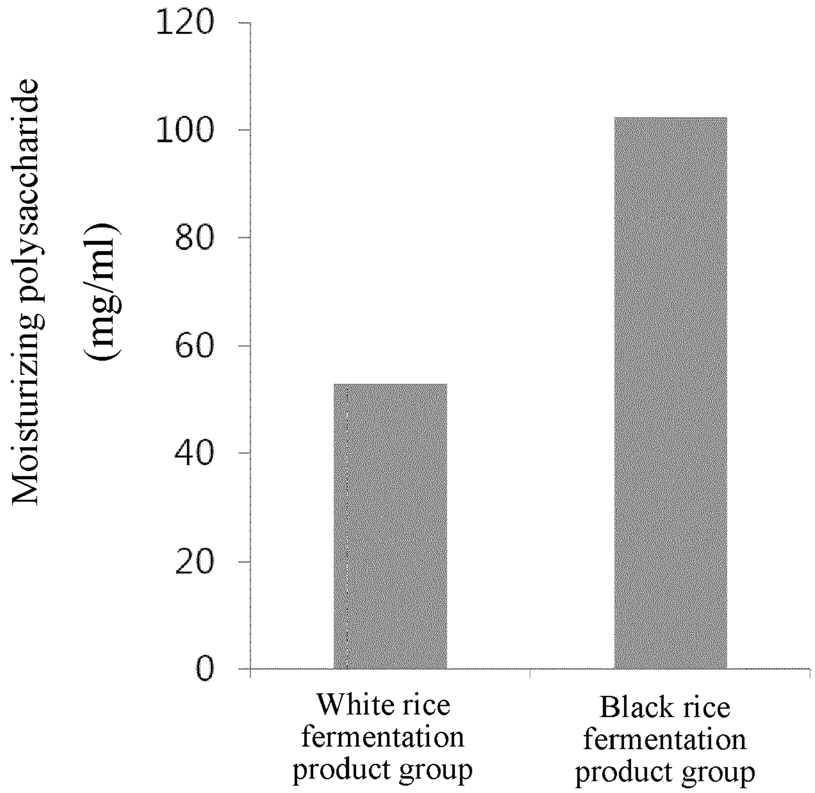
Moisturizing polysaccharide (mg/ml)
120
100
80
60
40
20
0
White rice fermentation product group
Black rice fermentation product group

FIG. 7

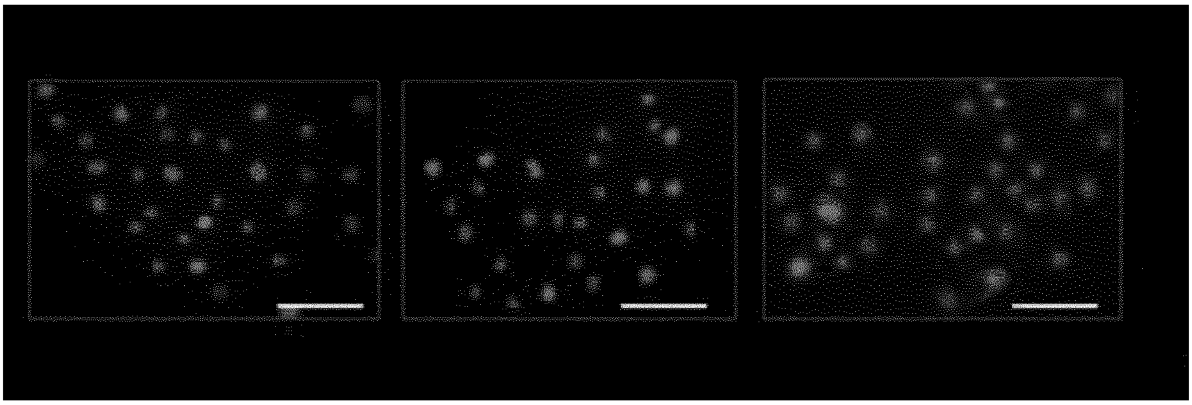

Control group | Black rice extract group | Black rice fermentation product group

FIG. 8

Control group | Black rice extract group | Black rice fermentation product group

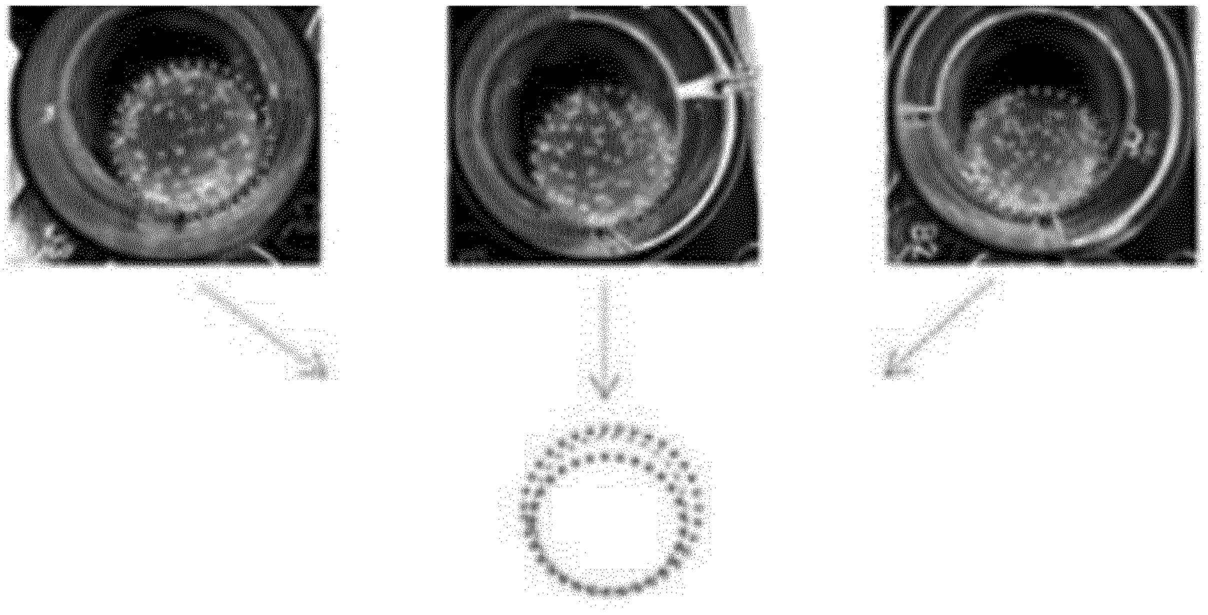

FIG. 9

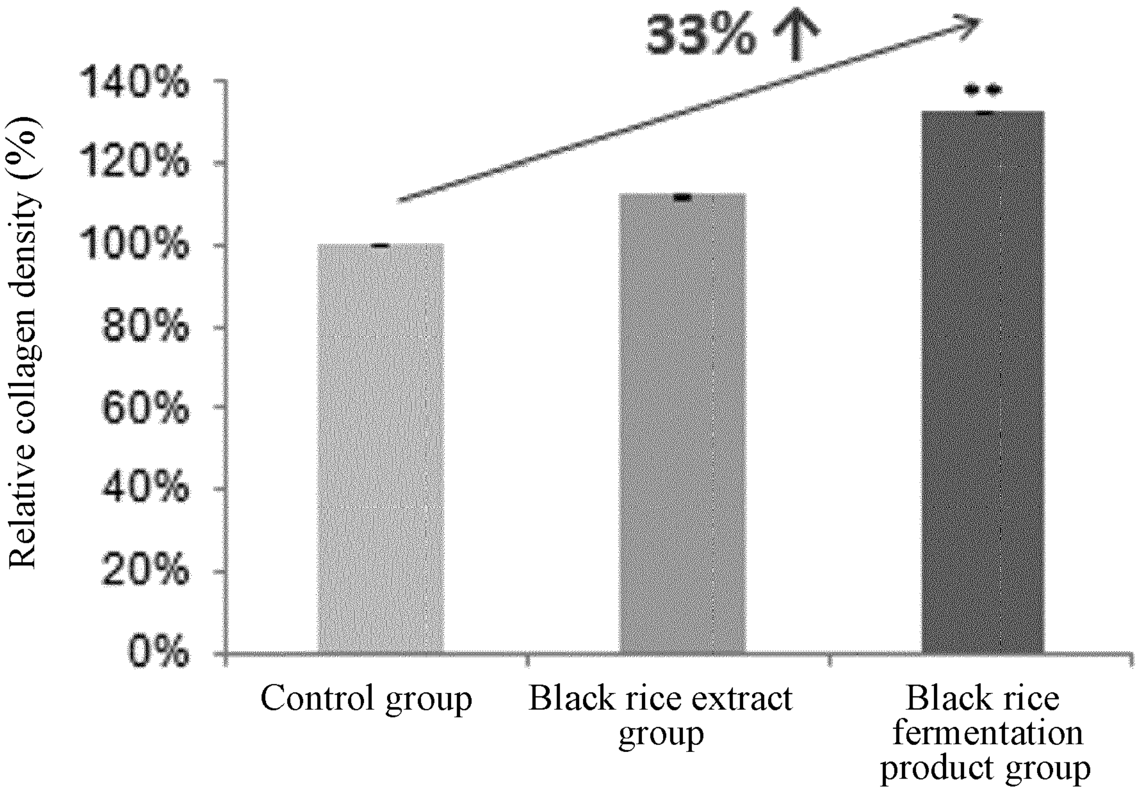
33% ↑
**
140%
120%
100%
80%
60%
40%
20%
0%
Relative collagen density (%)
Control group
Black rice extract group
Black rice fermentation product group

FIG. 10

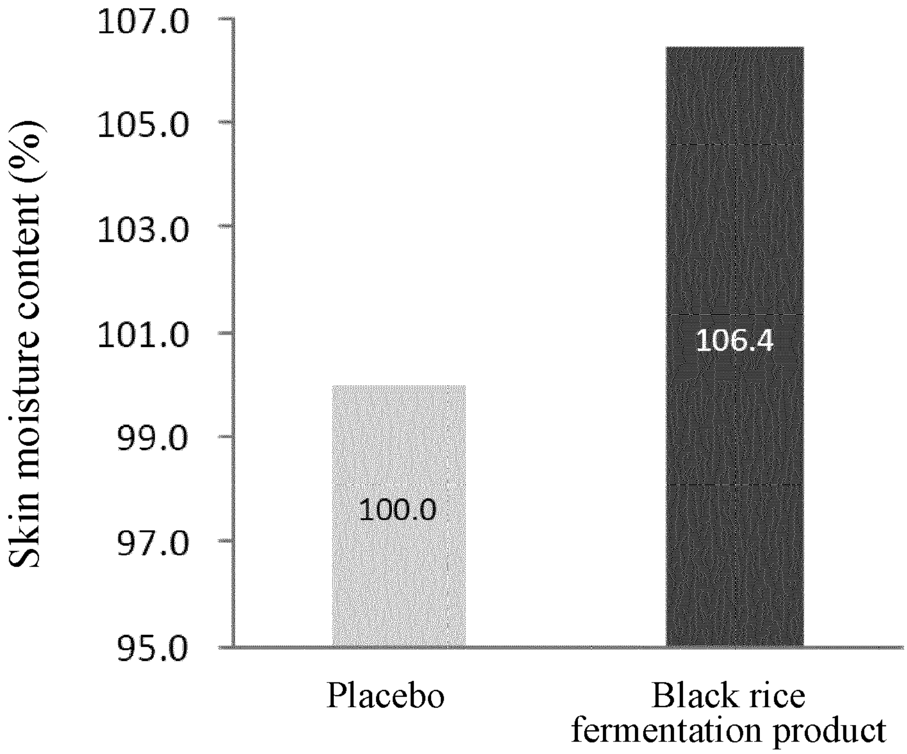
107.0
105.0
103.0
101.0
99.0
97.0
95.0
Skin moisture content (%)
100.0
106.4
Placebo
Black rice fermentation product

FIG. 11

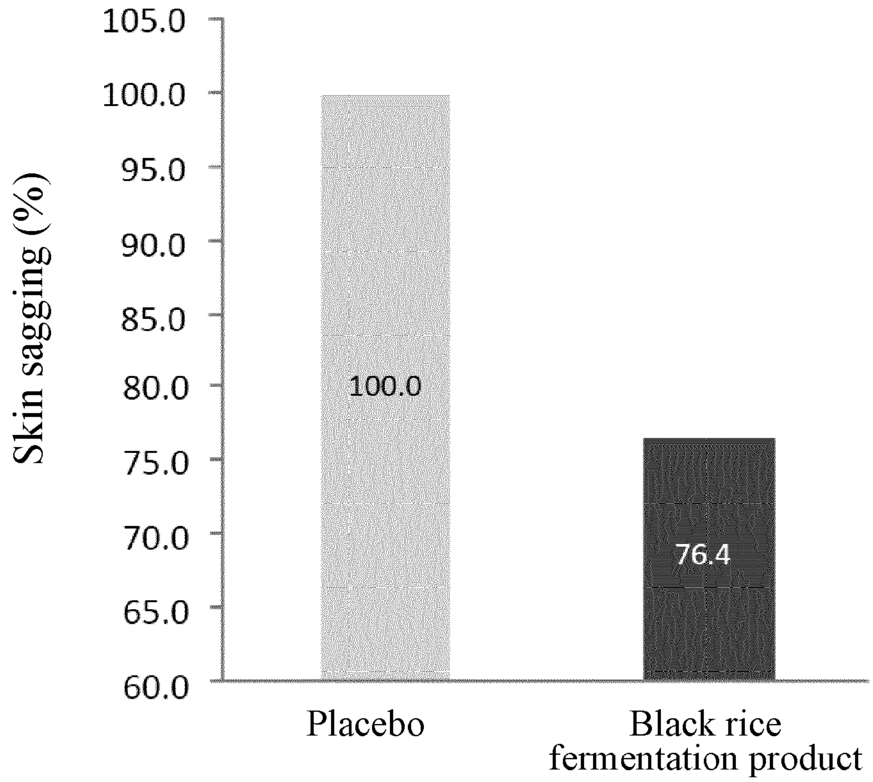
105.0
100.0
95.0
90.0
85.0
80.0
75.0
70.0
65.0
60.0
Skin sagging (%)
100.0
76.4
Placebo
Black rice fermentation product

FIG. 12

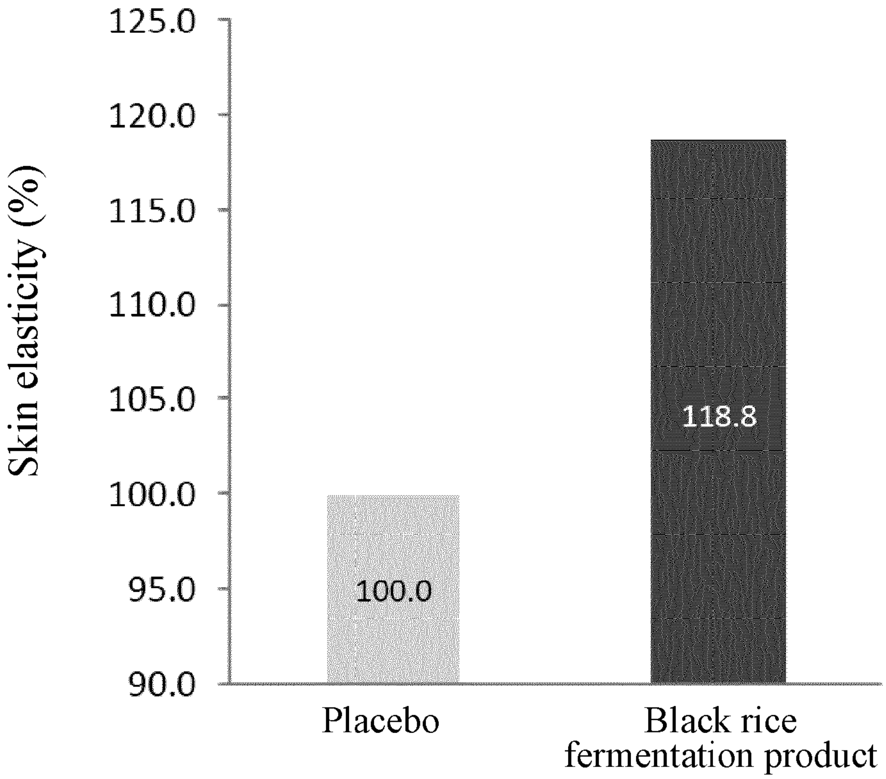
125.0
120.0
115.0
110.0
105.0
100.0
95.0
90.0
Skin elasticity (%)
100.0
118.8
Placebo
Black rice fermentation product

FIG. 13

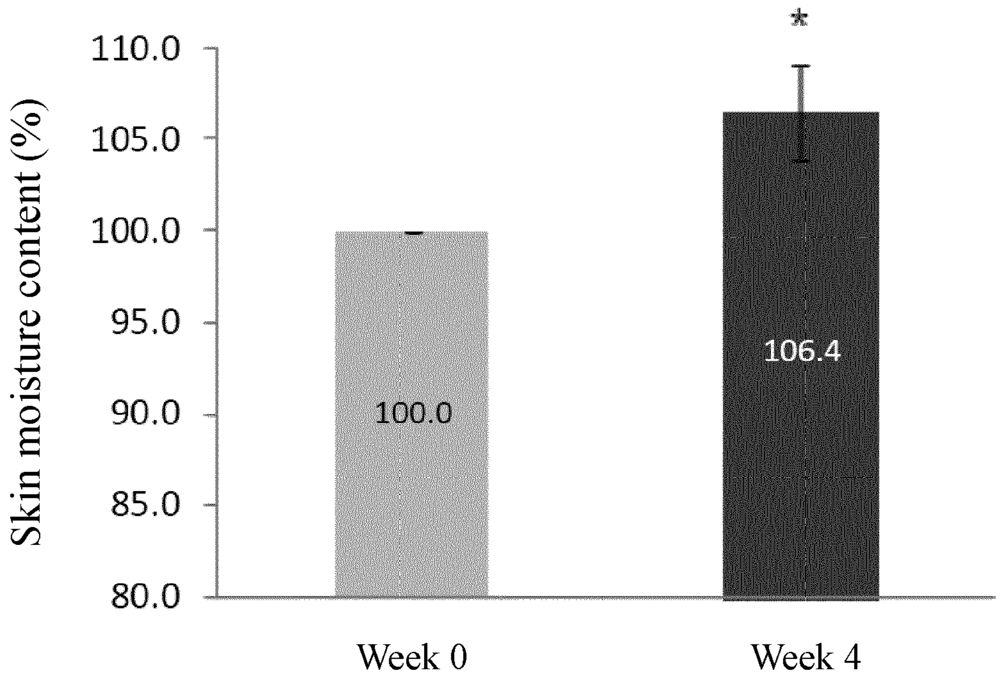
Skin moisture content (%)
110.0
105.0
100.0
95.0
90.0
85.0
80.0
*
100.0
106.4
Week 0
Week 4

FIG. 14

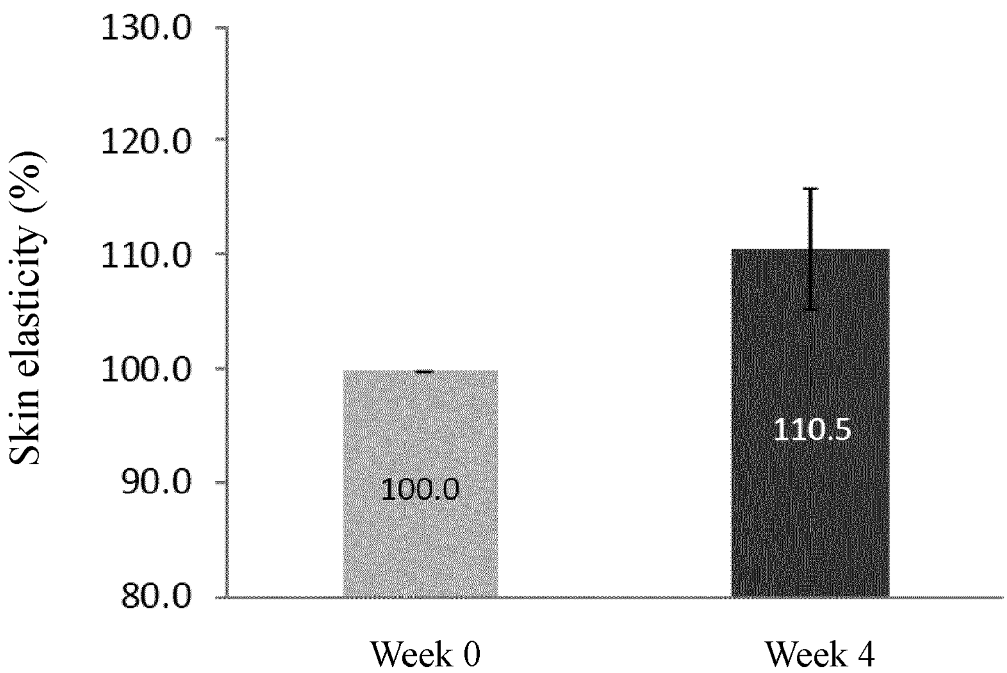
Skin elasticity (%)
130.0
120.0
110.0
100.0
90.0
80.0
100.0
110.5
Week 0
Week 4

FIG. 15

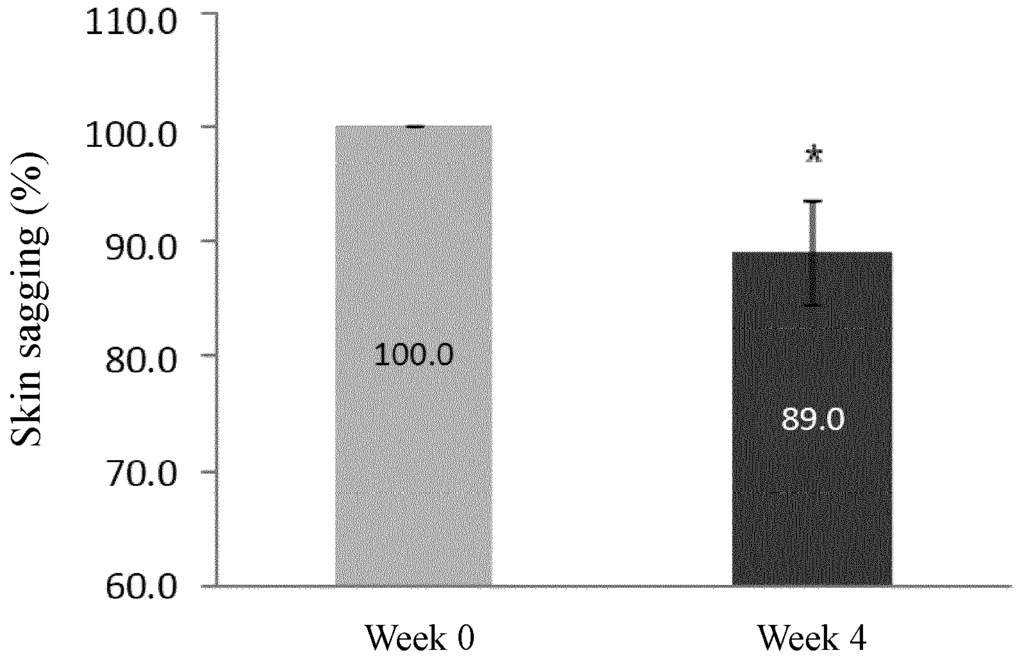
110.0
100.0
90.0
80.0
70.0
60.0
Skin sagging (%)
100.0
*
89.0
Week 0
Week 4

FIG. 16

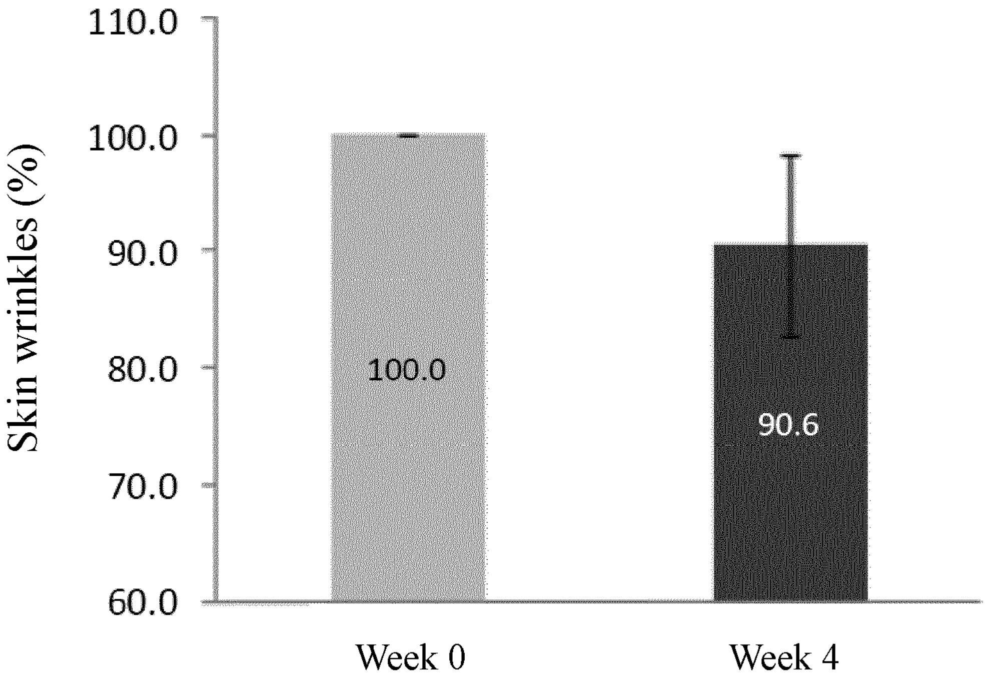
110.0
100.0
90.0
80.0
70.0
60.0
Skin wrinkles (%)
100.0
90.6
Week 0
Week 4

FIG. 17

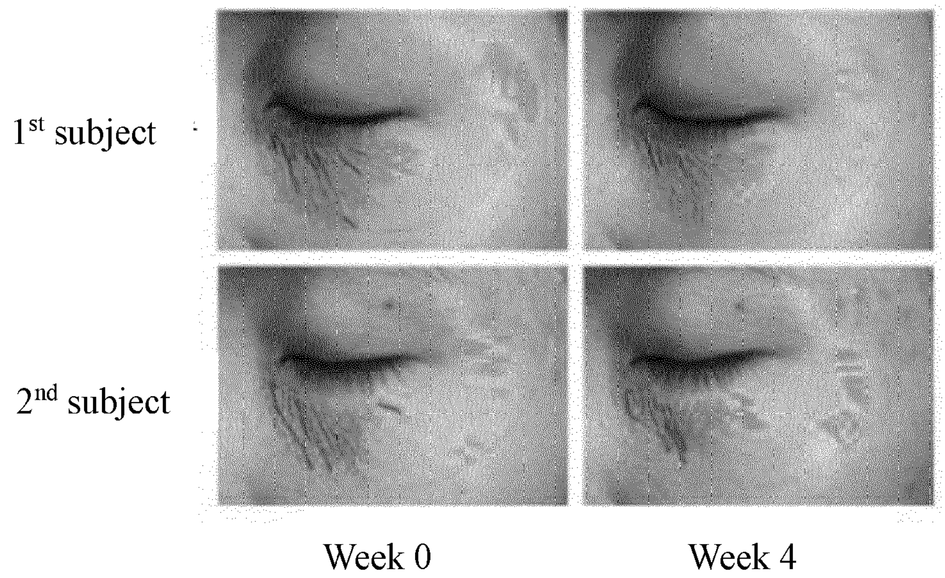
1st subject
2nd subject
Week 0
Week 4

FIG. 18

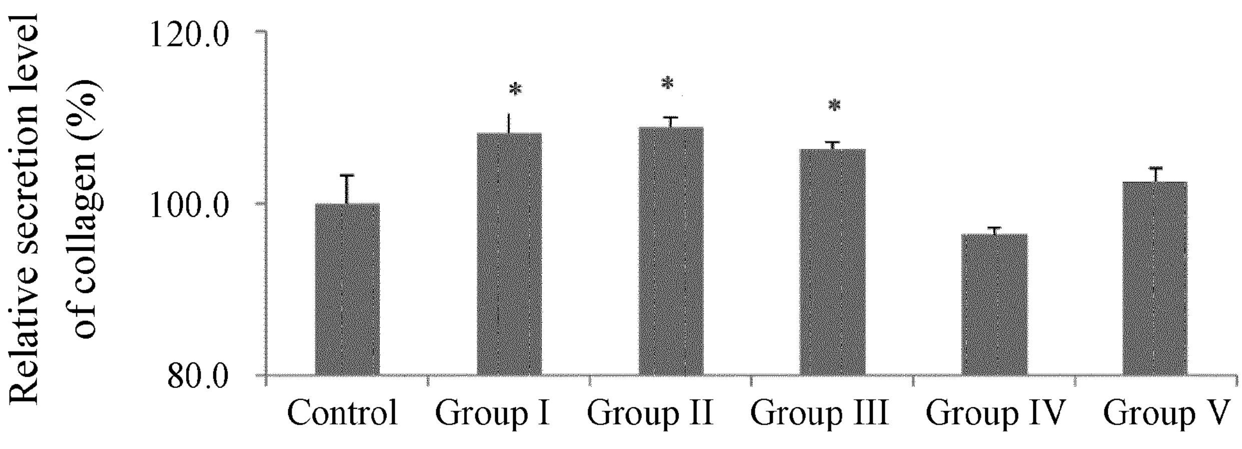
Relative secretion level of collagen (%)
120.0
100.0
80.0
*
*
*
Control
Group I
Group II
Group III
Group IV
Group V

FIG. 19

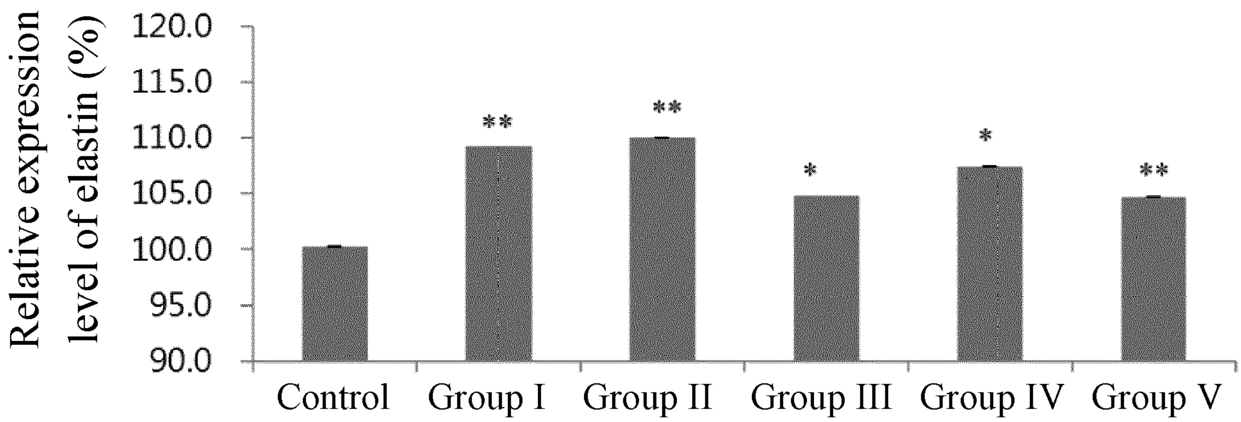
Relative expression level of elastin (%)
120.0
115.0
110.0
105.0
100.0
95.0
90.0
**
**
*
*
**
Control
Group I
Group II
Group III
Group IV
Group V

FIG. 20

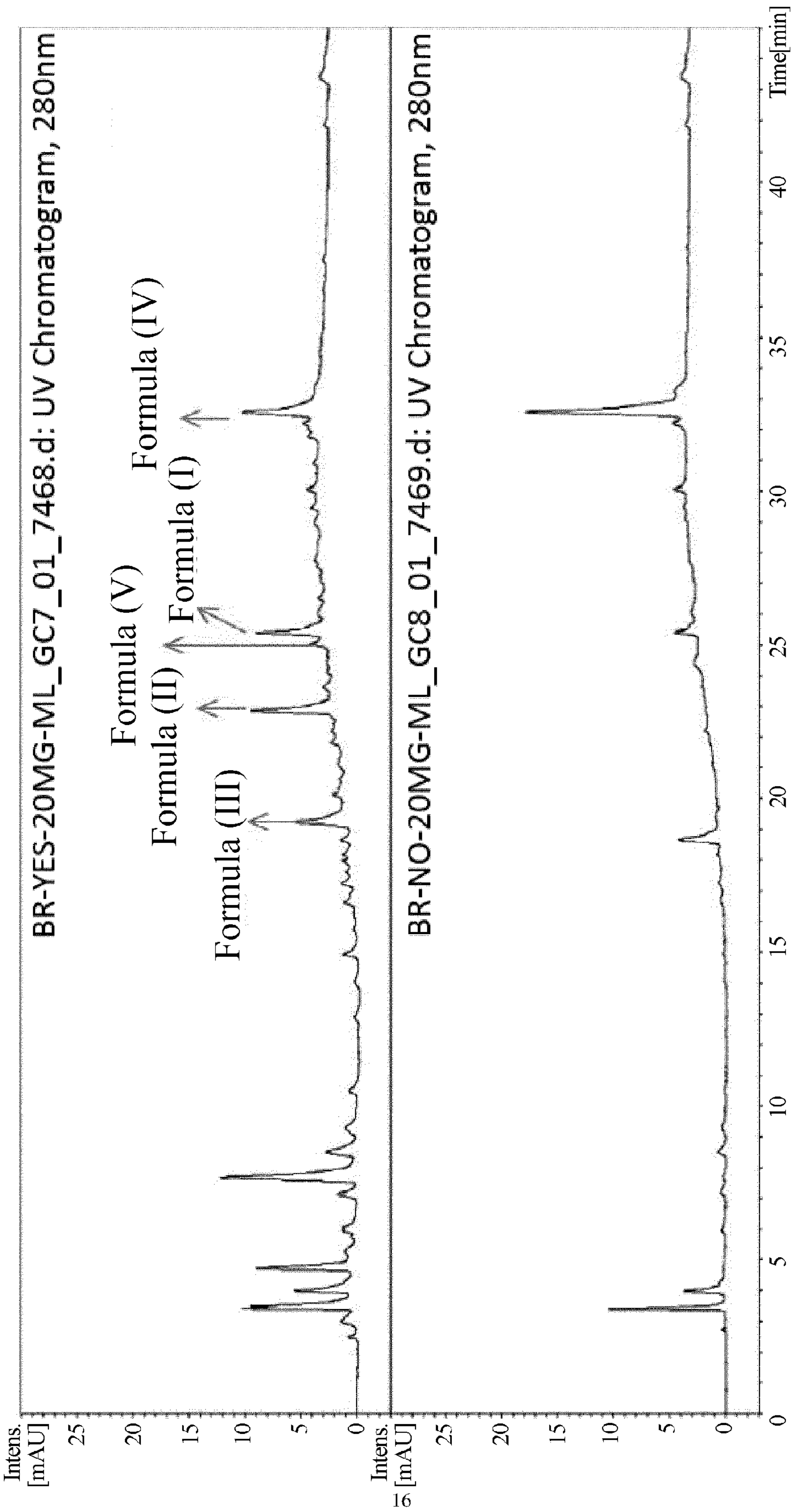
BR-YES-20MG-ML_GC7_01_7468.d: UV Chromatogram, 280nm
Formula (IV)
Formula (I)
Formula (V)
Formula (II)
Formula (III)
BR-NO-20MG-ML_GC8_01_7469.d: UV Chromatogram, 280nm
Intens. [mAU]
Intens. [mAU]
Time[min]
0
5
10
15
20
25
30
35
40
16

FIG. 21

Europäisches Patentamt
European Patent Office
Office européen des brevets

# EUROPEAN SEARCH REPORT

Application Number
EP 20 18 2011

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 101 701 827 B1 (NO YOUNG BAE [KR]; AGRICULTURAL CORP CO DAESANG [KR] ET AL.) 6 February 2017 (2017-02-06) * paragraphs [0001], [0002], [0022], [0023]; examples 1, 3 * | 1-15 | INV. A23L33/10 A23L33/105 A61P17/00 A61P17/18 A23L7/104 A61K8/368 A61Q19/08 A61K31/05 |
| X | KR 100 851 289 B1 (HANBIT FARMING ASS CO [KR]) 8 August 2008 (2008-08-08) * paragraphs [0015], [0016], [0022] - [0025], [0048] - [0052], [0060]; claims 1,2; example 4 * | 1-15 | |
| X | KR 2016 0014979 A (UNIV KOREA RES & BUS FOUND [KR]) 12 February 2016 (2016-02-12) * paragraphs [0003], [0015], [0022], [0090], [0096]; claims 1,10,11; examples 1,2 * | 6-15 | |
| X | WO 96/17589 A1 (KAO CORP) 13 June 1996 (1996-06-13) * page 1, paragraph 1; claims 1,7 * | 6-15 | TECHNICAL FIELDS SEARCHED (IPC) |
| X | JP 2016 079154 A (MARUZEN PHARMA) 16 May 2016 (2016-05-16) * paragraphs [0001], [0005], [0063], [0075]; claims 1-4,14 * | 6-15 | A23L A61P A61Q A61K |

The present search report has been drawn up for all claims

3

| Place of search | Date of completion of the search | Examiner |
|---|---|---|
| The Hague | 26 November 2020 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 18 2011

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2020

| Patent document cited in search report | Publication date | Patent family member(s) | Publication date |
|---|---|---|---|
| KR 101701827 B1 | 06-02-2017 | NONE | |
| KR 100851289 B1 | 08-08-2008 | NONE | |
| KR 20160014979 A | 12-02-2016 | NONE | |
| WO 9617589 A1 | 13-06-1996 | NONE | |
| JP 2016079154 A | 16-05-2016 | JP 6666650 B2 | 18-03-2020 |
| | | JP 2016079154 A | 16-05-2016 |

EPO FORM P0459

For more details about this annex : see Official Journal of the European Patent Office, No. 12/82